(19) Europäisches Patentamt
European Patent Office
Office européen des brevets

(11) **EP 4 139 041 B1**

(12) **EUROPEAN PATENT SPECIFICATION**

(45) Date of publication and mention
of the grant of the patent:
**25.02.2026 Bulletin 2026/09**

(21) Application number: **21719615.3**

(22) Date of filing: **16.04.2021**

(51) International Patent Classification (IPC):
**B01J 13/16** *(2006.01)*  **A61K 8/11** *(2006.01)*
**A61K 9/50** *(2006.01)*  **C11D 3/50** *(2006.01)*

(52) Cooperative Patent Classification (CPC):
**B01J 13/16; A61K 8/11; A61K 8/87; A61Q 13/00;
A61Q 19/00; C11D 3/3773; C11D 3/505;
C11D 17/0008;** A61K 9/5031; A61K 9/5089;
C11D 3/0015

(86) International application number:
**PCT/EP2021/059958**

(87) International publication number:
**WO 2021/213930 (28.10.2021 Gazette 2021/43)**

(54) **IMPROVEMENTS IN OR RELATING TO ORGANIC COMPOUNDS**

VERBESSERUNGEN AN ODER IM ZUSAMMENHANG MIT ORGANISCHEN VERBINDUNGEN

AMÉLIORATIONS APPORTÉES À DES COMPOSÉS ORGANIQUES OU SE RAPPORTANT À CEUX-CI

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.04.2020 GB 202005756**

(43) Date of publication of application:
**01.03.2023 Bulletin 2023/09**

(73) Proprietor: **Givaudan SA
1214 Vernier (CH)**

(72) Inventors:
• **RIFLADE, Benoit
33430 Bazas (CH)**
• **AUSSANT, Emmanuel
75011 Paris (FR)**

(74) Representative: **Global Patents
Givaudan SA
Grafenaustrasse 7
6300 Zug (CH)**

(56) References cited:
**WO-A1-2015/110568    WO-A1-2019/170528
US-A1- 2020 087 598**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention relates to a process for obtaining an encapsulated composition, to an encapsulated composition obtainable by this process, to a consumer product comprising such an encapsulated composition and to the use of such an encapsulated composition for obtaining a consumer product.

It is known to incorporate encapsulated functional materials in consumer products, such as household care, personal care and fabric care products. Functional materials include for example fragrances, cosmetic actives, biocides and substrate enhancers.

Microcapsules that are particularly suitable for delivery of such functional materials are core-shell microcapsules, wherein the core comprises the functional material and the shell is impervious or partially impervious to the functional material. Usually, these microcapsules are used in aqueous media and the encapsulated functional materials are hydrophobic. A broad selection of shell materials can be used, provided this shell material is impervious or partially impervious to the encapsulated functional material.

Among the functional materials, fragrances are encapsulated for a variety of reasons. Micro-capsules can isolate and protect the fragrances from external suspending media, such as consumer product bases, with which they may be incompatible or unstable in. They are also used to assist in the deposition of fragrance ingredients onto substrates, such as skin, hair, fabrics or hard household surfaces. They can also act as a means of controlling the spatio-temporal release of the fragrance.

Thermosetting resins are common encapsulating materials for encapsulating functional materials, especially volatile functional materials, such as fragrance ingredients. Core-shell microcapsules formed from aminoplast resins, polyurea resins, polyurethane resins, polyacrylate resins, and combinations thereof are generally quite resistant to fragrance leakage when dispersed in aqueous suspending media, even in surfactant-containing media. Furthermore, when incorporated into consumer products, such as laundry detergents or conditioners, they provide perfumery benefits that are unattainable if perfume is incorporated directly into those products.

WO 2008/098387 A1 discloses microcapsules having a shell formed from an aminoplast resin comprising a terpolymer comprising (a) moieties derived from at least one polyamine; (b) moieties derived from at least one aromatic polyol; and (c) alkylene or alkylenoxy moieties having 1 to 6 methylene units. These microcapsules are particularly impervious to fragrance ingredients, even over prolonged storage in products, but have a tendency to get a pink to brownish coloration during storage.

WO 2016/207180 A1 discloses microcapsules having a shell formed of an aminoplast resin comprising resorcinol and a cationic stabilizing polymer. These intrinsically cationic microcapsules are particularly impervious to fragrance ingredients, even over prolonged storage in products, but have a tendency to get a pink to brownish coloration during storage.

Documents WO 2015//110568 A1, US 2020/087598 A1, WO 2019/170528 A1 disclose core shell microcapsule compositions wherein a cationic polymer is added to the microcapsule dispersion post shell formation.

It is therefore a problem underlying the present invention to overcome the above-mentioned shortcomings in the prior art. In particular, it is a problem underlying the present invention to provide microcapsules showing improved stability with respect to coloration during storage, while still being stable with respect to leakage and performant in terms of olfactive benefits. These problems are solved by the subject-matter of the independent claims. In a first aspect, the present invention provides a process for obtaining an encapsulated composition comprising at least one cationic core-shell microcapsule. The at least one cationic core-shell microcapsule comprises a core comprising at least one functional material and a shell surrounding the core. The shell comprises a resin formed by reaction of at least one trifunctional araliphatic isocyanate with at least one partially alkylated amino-aldehyde pre-condensate. The process comprises the steps of:

a) Providing a core composition;
b) Providing an aqueous phase comprising the at least one partially alkylated amino-aldehyde pre-condensate and a cationic stabilizing polymer;
c) Emulsifying the core composition provided in step a) with the aqueous phase provided in step b) in order to obtain an emulsion of core composition droplets having a volume average size of 1 to 100 $\mu$m, preferably 5 to 50 $\mu$m, even more preferably 7 to 20 $\mu$m;
d) Heating the emulsion obtained in step c) to a temperature of from 70 to 95 °C, preferably from 80 to 90 °C; preferably at a pH of 2.0 to 4.5, more preferably of 2.5 to 4.0, still more preferably of 3.0 to 3.8, even more preferably of 3.4 to 3.6, in order to obtain core-shell microcapsules;
e) Optionally: Adding a formaldehyde scavenging agent; and
f) Letting the mixture obtained in step d) or step e) cool to room temperature, in order to obtain a slurry of microcapsules.

The at least one trifunctional araliphatic isocyanate is added to the aqueous phase or the emulsion before, during or after step c).

In the present context, it has been found that the problem of discoloration mentioned hereinabove can be solved by replacing the moieties of the copolymer derived from the at least one aromatic alcohol by moieties derived from at least one araliphatic isocyanate.

Furthermore, it has been found that the method of addition of the at least one araliphatic polyisocyanate in the microencapsulation process is relevant for controlling the imperviousness of the encapsulating material with respect to fragrance leakage, while still maintaining, and even improving, the olfactive performance of the encapsulated composition. More specifically, adding the trifunctional araliphatic isocyanate to the aqueous phase or to the emulsion does significantly improve the imperviousness of aminoplast microcapsule shells, compared to adding poly-functional araliphatic isocyanate to the core composition.

In preferred embodiments, the at least one araliphatic trifunctional araliphatic isocyanate is an adduct of an aliphatic triol with three araliphatic di-isocyanates.

Without being bound by any theory, the applicant believes that araliphatic isocyanate groups have the advantage of possessing an intermediate reactivity compared to the highly reactive aromatic isocyanates and the less reactive aliphatic isocyanate.

In further preferred embodiments, the at least one trifunctional araliphatic isocyanate is an adduct of 2-ethylpropane-1,2,3-triol or 2-ethyl-2-(hydroxymethyl)propane-1,3-diol with a compound selected from the group consisting of 1-isocyanato-2-(isocyanatomethyl)benzene, 1-isocyanato-3-(isocyanatomethyl)-benzene and 1-isocyanato-4-(isocyanatomethyl)benzene.

In a particularly preferred embodiment, the at least one trifunctional araliphatic isocyanate is an adduct of 2-ethylpropane-1,2,3-triol with 1-isocyanato-3-(isocyanatomethyl)benzene.

Adducts of 2-ethylpropane-1,2,3-triol with 1-isocyanato-3-(isocyanatomethyl)-benzene are available commercially under the trade names Takenate D110-N (ex Mitsui Chemicals) or Quix 175 (ex Covestro).

Alkylated amino-aldehyde pre-condensates that are suitable for the sake of the present invention are well-known to the art. In preferred embodiments of the invention, the at least one partially alkylated amino-aldehyde precondensate is selected from the group consisting of partially methylated poly-methylol-1,3,5-triamino-2,4,6-triazine pre-condensates, partially methylated poly-methylol-urea, partially methylated poly-methylol-benzoguanamine and partially methylated poly-methylolglycouril; more preferably partially methylated poly-methylol-1,3,5-triamino-2,4,6-triazine pre-condensates.

A particularly preferred partially methylated poly-methylol-1,3,5-triamino-2,4,6-triazine pre-condensate is obtainable by reacting melamine, formaldehyde and methanol and wherein the molar ratio of melamine to formaldehyde is from 1:3 to 1:6 and the molar ratio of melamine to alkylol is 1:2 to 1:4.

Such a pre-condensate is available under the name Luracoll SD, ex BASF, has been especially optimized for forming encapsulating aminoplast materials at the oil/water interface, which is an advantage.

In preferred embodiments of the present invention, the weight ratio of the at least one trifunctional araliphatic isocyanate to the at least one partially alkylated amino-aldehyde pre-condensate is from 0.25 to 0.50, more preferably from 0.3 to 0.4. Without being bound by any theory, it is assumed that this range coincides with the optimal ratio between the available amine functions of the poly-methylol-1,3,5-triamino-2,4,6-triazine pre-condensate and the available isocyanate functions of the trifunctional araliphatic isocyanate.

Cationic stabilizing polymers that are particularly suitable for the sake of the present invention maybe of synthetic origin or derived from nature. However, copolymers of synthetic origin are preferred because of their enhanced compatibility with the oil/water interface and the encapsulating aminoplast material.

The cationic polymer employed as a stabilizer may be an ampholytic polymer and more particularly an ampholytic co-polymercomprising cationicquaternary ammonium groups, which have the advantage of having a pH-independent positive charge.

In a particular way, the cationic group is derived from a monomer, which is preferably selected from the group consisting of quaternized dimethylaminoethyl acrylate (ADAME), quaternized dimethylaminoethyl methacrylate (MADAME), dimethyldiallyl ammonium chloride (DADMAC), acrylamidopropyltrimethylammonium chloride (APTAC) and methacrylamidopropyltrimethylammonium chloride (MAPTAC). The most preferred cationic monomer is MAPTAC.

The ampholytic copolymer may comprise anionic groups or groups that may be anionic under certain pH conditions, such as carboxylate, sulfate, sulfonate and phosphate. In a particular way, the anionic group is ed ns is derived from a monomer selected from the group consisting of acrylic based monomers, include acrylic acid, methacrylic acid, itaconic acid, crotonic acid, maleic acid, fumaric acid and strong-acid monomers, for example monomers with a sulfonic or a phosphonic acid-type function such as 2-acrylamido- 2-methylpropane sulfonic acid, vinylsulfonic acid, vinylphosphonic acid, allylsulfonic acid, allylphosphonic acid, styrene sulfonic acid. The acrylic based monomer may also be any water-soluble salts of these monomers, wherein the salt is a salt of an alkali metal, an alkaline-earth metal or an ammonium. The most preferred acrylic based monomer is acrylic acid, methacrylic acid, or a water soluble salt thereof.

The ampholytic polymer contains more cationic groups than groups that can form anions, and as such, is characterized in that it has a net positive charge over the full accessible pH range, preferably between pH=1 and pH=13.

The ampholytic copolymer may also comprise non-ionic moieties that are preferably derived from a non-ionic monomer selected from the group consisting of water-soluble vinyl monomers, and more particularly acrylamide, methacrylamide, N-isopropylacrylamide, N,N-dimethylacrylamide, N-methylolacrylamide. N-vinylformamide, N-vinyl acetamide, N-vinylpyridine and/or N-vinylpyrrolidone can also be used. The preferred non-ionic monomer is acrylamide.

Preferably, the copolymer has a molecular weight of at least 100'000 g/mol, and more particularly at least 500'000 g/mol.

In preferred embodiments of the present invention, the cationic stabilizing polymer is a cationic ampholyte copolymer obtainable by copolymerizing methacrylamidopropyltrimethylammonium chloride, acrylic acid or methacrylic acid and, optionally, acrylamide.

In particularly preferred embodiments, the cationic stabilizing copolymer is a copolymer of 2 to 99 mol %, more particularly 30 to 95 mol %, still more particularly 60 to 90 mol %, of methacrylamidopropyltrimethylammonium chloride (MAPTAC); and 1 to 98 mol %, more particularly 5 to 70 mol %, still more particularly 10 to 40 mol %, of acrylic acid, and 0 to 50 mol %, more particularly 0.1 to 5 mol %, of acrylamide.

The amount of ampholytic polymer that may be employed in an encapsulated perfume composition according to the present invention may be from 1 to 20 wt.-% and more particularly 2 to 10 wt.-% based on the weight of the composition. In particular, the ampholyte copolymer improves (i) the stability of the emulsion in which the microcapsules are formed, (ii) the control of the thickness of the microcapsules' shell and (iii) the prevention of the formation of agglomerates of microcapsules.

In particular embodiments of the present invention, the process may additionally comprise the step of adding, between step d) and step f), preferably between step e) and step f), a suspending agent.

Such suspending agents may prevent the formation of microcapsule agglomerates and/or prevent the microcapsule to cream or sediment.

Suspending agents that are particularly useful for the sake of the present invention include starch and starch derivatives, such as modified starch, dextrin, xanthan gum, gum tragacanth, gum karaya, guar gum; cellulose and cellulose derivatives, such as hydroxyethyl cellulose, hydroxyethyl cellulose/lauryl-dimethylammoniumepoxy condensate, hydroxypopyl cellulose, cationic cellulose (for example Polyquaternium-4), cellulose gum; carrageenan; agar-agar; pectines and pectic acid; gelatine; protein hydrolysates; polymer and copolymers of vinyl and allyl monomers, such as polyvinylpyrrolidone; poly(vinyl pyrrolidone-co-vinylacetate); poly(vinyl alcohol-co-vinyl acetate), more particularly hydrolyzed polyvinylacetates having a degree of hydrolysis between 85 and 92%; vinyl ester homopolymers and copolymers, such as vinyl acetate, vinyl pivalate, vinyl versatate; poly(vinyl methyl ether), poly(vinyl alkyl amines), such as poylvinylmethylamine; quaternized polyvinyl alkyl amines, vinyl pyridine and quaternized vinyl pyridine, vinyl imidazoline, vinyl imidazole, vinyl imidazolinium, dimethyldiallyl ammonium chloride; polyamines and polyimines; ethoxylated polyamines; polymers, (meth)acrylamides; N-alkyl (meth)acrylamides, such as N,N-dimethylaminoalkyl methacrylate; quaternized N-alkyl (meth)acrylamides, such as methacrylamidopropyl-trimethylammonium chloride, acrylamidoe-thyltrimonium chloride, acrylamidolauryltrimethyl-ammonium chloride and 2-(acryloyloxy)-N,N,N-trimethylethanaminium chloride; poly(alkyleneoxide); polyurethanes and polyureas, such as cationic non-ionic and amphoteric polyurethanes and polyureas; mixed copolymers thereof; and mixture thereof.

In preferred embodiments of the invention, the suspending agent is a cationic polymer, preferably a cationic copolymer.

In preferred embodiments of the present invention, the process additionally comprises the step of adding at least one preservative to the mixture obtained in step d) or to the slurry of microcapsules obtained in step f).

Suitable preservatives include quaternary compounds, biguanide compounds poylaminopropyl biguanidine, Hexetidine, para-chloro-meta-cresol, methenamine, 3-Bis(hydroxymethyl)-5,5-dimethylimidazolidine-2,4-dione, Quaternium-15, benzoic acid, salicylic acid, undec-10-enoic acid, formic acid, biphenyl-2-ol and their salts, 4-hydroxybenzoic acid and its esters and salts; sorbic acid and its salts, Isothiazolinones, Bronopol (2-Bromo-2-nitro-1,3-propanediol), 5-bromo-5-nitro-1,3-dioxane, Thiabendazone, Benzimidazole carbamate, Triclocarban; 3-Iodo-2-propynylbutylcarbamate, Thiomersal; Triclosan, dichlorobenzyl alcohol, chloroxylenol, imidazolidinyl urea, phenoxyethanol, benzyl alcohol; caprilyl glycol and mixture thereof.

The at least one functional material comprised in the core composition is typically hydrophobic or has a limited solubility in water, for example less than 2.5 g per 100 g of water, preferably less than 1 g per 100 g of water, still more preferably less than 0.1 g per 100 g of water. Preferably, the functional material is liquid or soluble in apolar solvents, such as oils, so that, when admixed with water, the core composition and the aqueous phase form two separate phases. Preferably, the at least one functional material has a calculated octanol/water partition coefficient (ClogP) of 1.5 or more, more preferably 3 or more. Preferably, the ClogP of the functional cosmetic ingredients is from about 2 to about 7.

Preferably, the amount of core composition is lower than the amount of aqueous phase, so that, when emulsified with the aqueous phase in step c) of the process according to the present invention, the core composition forms a dispersed phase in the aqueous phase, generally in the form of droplets, which are stabilized by the cationic stabilizing polymer.

These stabilized droplets act as templates on which the microencapsulation may take place.

In preferred embodiments of the present invention, the core composition comprises at least one functional material may selected from group consisting of fragrance ingredients, cosmetic ingredients, biologically active ingredients, and substrate enhancers.

In preferred embodiments of the present invention, the core composition comprises at least one perfume ingredient. A comprehensive list of perfume ingredients that may be encapsulated in accordance with the present invention may be found in the perfumery literature, for example "Perfume & Flavor Chemicals", S. Arctander (Allured Publishing, 1994). Encapsulated perfume according to the present invention preferably comprise perfume ingredients selected from ADOXAL (2,6,10-trimethylundec-9-enal); AGRUMEX (2-(tert-butyl)cyclohexyl acetate); ALDEHYDE C 10 DECYLIC (decanal); ALDEHYDE C 11 MOA (2-methyldecanal); ALDEHYDE C 11 UNDECYLENIC (undec-10-enal); ALDEHYDE C 110 UNDECYLIC (undecanal); ALDEHYDE C 12 LAURIC (dodecanal); ALDEHYDE C 12 MNA PURE (2-methylundecanal); ALDEHYDE ISO C 11 ((E)-undec-9-enal); ALDEHYDE MANDARINE 10%/TEC ((E)-dodec-2-enal); ALLYL AMYL GLYCOLATE (allyl 2-(isopentyloxy)acetate); ALLYL CYCLOHEXYL PROPIONATE (allyl 3-cyclohexylpropanoate); ALLYL OENANTHATE (allyl heptanoate); AMBER CORE (1-((2-(tert-butyl)cyclohexyl)oxy)butan-2-ol); AMBERMAX (1,3,4,5,6,7-hexahydro-.beta.,1,1,5,5-pentamethyl-2H-2,4a-Methanonaphthalene-8-ethanol); AMYL SALICYLATE (pentyl 2-hydroxybenzoate); APHERMATE (1-(3,3-dimethylcyclohexyl)ethyl formate); BELAMBRE ((1R,2S,4R)-2'-isopropyl-1,7,7-trimethylspiro[bicyclo[2.2.1]heptane-2,4'-[1,3]dioxane]); BIGARYL (8-(sec-butyl)-5,6,7,8-tetrahydroquinoline); BOISAMBRENE FORTE ((ethoxymethoxy)-cyclododecane); BOISIRIS ((1S,2R,5R)-2-ethoxy-2,6,6-trimethyl-9-methylene-bicyclo[3.3.1]nonane); BORNYL ACETATE ((2S,4S)-1,7,7-trimethyl-bicyclo[2.2.1]-heptan-2-yl acetate); BUTYL BUTYRO LACTATE (1-butoxy-1-oxopropan-2-yl butyrate); BUTYL CYCLOHEXYL ACETATE PARA (4-(tert-butyl)cyclohexyl acetate); CARYOPHYLLENE ((Z)-4,11,11-trimethyl-8-methylenebicyclo[7.2.0]-undec-4-ene); CASHMERAN (1,1,2,3,3-pentamethyl-2,3,6,7-tetrahydro-1H-inden-4(5H)-one); CASSYRANE (5-tert-butyl-2-methyl-5-propyl-2H-furan); CITRAL ((E)-3,7-dimethylocta-2,6-dienal); CITRAL LEMAROME N ((E)-3,7-dimethylocta-2,6-dienal); CITRATHAL R ((Z)-1,1-diethoxy-3,7-dimethylocta-2,6-diene); CITRONELLAL (3,7-dimethyloct-6-enal); CITRONELLOL (3,7-dimethyloct-6-en-1-ol); CITRONELLYL ACETATE (3,7-dimethyloct-6-en-1-yl acetate); CITRONELLYL FORMATE (3,7-dimethyloct-6-en-1-yl formate); CITRONELLYL NITRILE (3,7-dimethyloct-6-enenitrile); CITRONELLYL PROPIONATE (3,7-dimethyloct-6-en-1-yl propionate); CLONAL (dodecanenitrile); CORANOL (4-cyclohexyl-2-methylbutan-2-ol); COSMONE ((Z)-3-methylcyclotetradec-5-enone); CYCLAMEN ALDEHYDE (3-(4-isopropylphenyl)-2-methylpropanal); CYCLOGALBANATE (allyl 2-(cyclohexyloxy)acetate); CYCLOHEXYL SALICYLATE (cyclohexyl 2-hydroxybenzoate); CYCLOMYRAL (8,8-dimethyl-1,2,3,4,5,6,7,8-octahydronaphthalene-2-carbaldehyde); DAMASCENONE ((E)-1-(2,6,6-trimethylcyclohexa-1,3-dien-1-yl)but-2-en-1-one); DAMASCONE ALPHA ((E)-1-(2,6,6-trimethylcyclohex-2-en-1-yl)but-2-en-1-one); DAMASCONE DELTA ((E)-1-(2,6,6-trimethylcyclohex-3-en-1-yl)but-2-en-1-one); DECENAL-4-TRANS ((E)-dec-4-enal); DELPHONE (2-pentylcyclopentanone); DIHYDRO ANETHOLE (propanedioic acid 1-(1-(3,3-dimethylcyclohexyl)ethyl) 3-ethyl ester); DIHYDRO JASMONE (3-methyl-2-pentylcyclopent-2-enone); DIMETHYL BENZYL CARBINOL (2-methyl-1-phenylpropan-2-ol); DIMETHYL BENZYL CARBINYL ACETATE (2-methyl-1-phenylpropan-2-yl acetate); DIMETHYL BENZYL CARBINYL BUTYRATE (2-methyl-1-phenylpropan-2-yl butyrate); DIMETHYL OCTENONE (4,7-dimethyloct-6-en-3-one); DIMETOL (2,6-dimethylheptan-2-ol); DIPENTENE (1-methyl-4-(prop-1-en-2-yl)cyclohex-1-ene); DUPICAL ((E)-4-((3aS,7aS)-hexahydro-1H-4,7-methanoinden-5(6H)-ylidene)butanal); EBANOL ((E)-3-methyl-5-(2,2,3-trimethylcyclopent-3-en-1-yl)pent-4-en-2-ol); ETHYL CAPROATE (ethyl hexanoate); ETHYL CAPRYLATE (ethyl octanoate); ETHYL LINALOOL ((E)-3,7-dimethylnona-1,6-dien-3-ol); ETHYL LINALYL ACETATE ((Z)-3,7-dimethylnona-1,6-dien-3-yl acetate); ETHYL OENANTHATE (ethyl heptanoate); ETHYL SAFRANATE (ethyl 2,6,6-trimethylcyclohexa-1,3-diene-1-carboxylate); EUCALYPTOL ((1s,4s)-1,3,3-trimethyl-2-oxabicyclo[2.2.2]octane); FENCHYL ACETATE ((2S)-1,3,3-trimethylbicyclo[2.2.1]heptan-2-yl acetate); FENCHYL ALCOHOL ((1S,2R,4R)-1,3,3-trimethylbicyclo[2.2.1]heptan-2-ol); FIXOLIDE (1-(3,5,5,6,8,8-hexamethyl-5,6,7,8-tetrahydronaphthalen-2-yl)ethanone); FLOR-ALOZONE (3-(4-ethylphenyl)-2,2-dimethylpropanal); FLORHYDRAL (3-(3-isopropylphenyl)butanal); FLOROCYCLENE ((3aR,6S,7aS)-3a,4,5,6,7,7a-hexahydro-1H-4,7-methanoinden-6-yl propionate); FLOROPAL (2,4,6-trimethyl-4-phenyl-1,3-dioxane); FRESKOMENTHE (2-(sec-butyl)cyclohexanone); FRUITATE ((3aS,4S,7R,7aS)-ethyl octahydro-1H-4,7-methanoindene-3a-carboxylate); FRUTONILE (2-methyldecanenitrile); GALBANONE PURE (1-(3,3-dimethylcyclohex-1-en-1-yl)pent-4-en-1-one); GARDOCYCLENE ((3aR,6S,7aS)-3a,4,5,6,7,7a-hexahydro-1H-4,7-methanoinden-6-yl isobutyrate); GERANIOL ((E)-3,7-dimethylocta-2,6-dien-1-ol); GERANYL ACETATE SYNTHETIC ((E)-3,7-dimethylocta-2,6-dien-1-yl acetate); GERANYL ISOBUTYRATE ((E)-3,7-dimethylocta-2,6-dien-1-yl isobutyrate); GIVESCONE (ethyl 2-ethyl-6,6-dimethylcyclohex-2-enecarboxylate); HABANOLIDE ((E)-oxacyclohexadec-12-en-2-one); HEDIONE (methyl 3-oxo-2-pentylcyclopentaneacetate); HERBANATE ((2S)-ethyl 3-isopropylbicyclo[2.2.1]hept-5-ene-2-carboxylate); HEXENYL-3-CIS BUTYRATE ((Z)-hex-3-en-1-yl butyrate); HEXYL CINNAMIC ALDEHYDE ((E)-2-benzylideneoctanal); HEXYL ISOBUTYRATE (hexyl isobutyrate); HEXYL SALICYLATE (hexyl 2-hydroxybenzoate); INDOFLOR (4,4a,5,9b-tetrahydroindeno[1,2-d][1,3]dioxine); IONONE BETA ((E)-4-(2,6,6-trimethylcyclohex-1-en-1-yl)but-3-en-2-one); IRISONE ALPHA ((E)-4-(2,6,6-trimethylcyclohex-2-en-1-yl)but-3-en-2-one); IRONE ALPHA ((E)-4-(2,5,6,6-tetramethylcyclohex-2-en-1-yl)but-3-en-2-one); ISO E SUPER (1-(2,3,8,8-tetramethyl-1,2,3,4,5,6,7,8-octahydronaphthalen-2-yl)ethanone); ISOCYCLO-CITRAL (2,4,6-trimethylcyclohex-3-enecarbaldehyde); ISONONYL ACETATE (3,5,5-trimethylhexyl acetate); ISOPROPYL METHYL-2-BUTYRATE (isopropyl 2-methyl butanoate); ISORALDEINE 70 ((E)-3-methyl-4-(2,6,6-trimethylcyclohex-2-en-1-yl)but-3-en-2-one); JASMACYCLENE ((3aR,6S,7aS)-3a,4,5,6,7,7a-hexahydro-1H-4,7-methanoinden-6-yl acetate); JASMONE CIS ((Z)-3-methyl-2-(pent-2-en-1-yl)cyclopent-2-enone); KARANAL (5-(sec-butyl)-2-(2,4-dimethylcyclohex-3-en-1-yl)-5-methyl-1,3-dioxane); KOAVONE ((Z)-3,4,5,6,6-pentamethylhept-3-en-2-one); LEAF ACETAL ((Z)-1-(1-ethoxyethoxy)hex-3-ene); LEMONILE ((2E,6Z)-3,7-dimethylnona-2,6-dienenitrile); LIFFAROME GIV ((Z)-hex-3-en-1-yl methyl carbonate); LILIAL (3-(4-(tert-butyl)phenyl)-2-methylpropanal); LINALOOL (3,7-dimethylocta-1,6-dien-3-ol); LINALYL ACETATE (3,7-dimethylocta-1,6-dien-3-yl acetate); MAHONIAL ((4E)-9-hydroxy-5,9-dimethyl-4-decenal); MALTYL ISOBUTYRATE (2-methyl-4-oxo-4H-pyran-3-yl isobutyrate); MANZANATE (ethyl 2-methylpentanoate); MELONAL (2,6-dimethylhept-5-enal); MENTHOL (2-isopropyl-5-methylcyclohexanol); MENTHONE (2-isopropyl-5-methylcyclohexanone); METHYL CEDRYL KETONE (1-((1S,8aS)-1,4,4,6-tetramethyl-2,3,3a,4,5,8-hexahydro-1H-5,8a-methanoazulen-7-yl)ethanone); METHYL NONYL KETONE EXTRA (undecan-2-one); METHYL OCTYNE CARBONATE (methyl non-2-ynoate); METHYL PAMPLEMOUSSE (6,6-dimethoxy-2,5,5-trimethylhex-2-ene); MYRALDENE (4-(4-methylpent-3-en-1-yl)cyclohex-3-enecarbaldehyde); NECTARYL (2-(2-(4-methylcyclohex-3-en-1-yl)propyl)cyclopentanone); NEOBERGAMATE FORTE (2-methyl-6-methyleneoct-7-en-2-yl acetate); NEOFOLIONE ((E)-methyl non-2-enoate); NEROLIDYLE ((Z)-3,7,11-trimethyldodeca-1,6,10-trien-3-yl acetate); NERYL ACETATE HC ((Z)-3,7-dimethylocta-2,6-dien-1-yl acetate); NONADYL (6,8-dimethylnonan-2-ol); NONENAL-6-CIS ((Z)-non-6-enal); NYMPHEAL (3-(4-isobutyl-2-methylphenyl)propanal); ORIVONE (4-(tert-pentyl)cyclohexanone); PARADISAMIDE (2-ethyl-N-methyl-N-(m-tolyl) butanamide); PELARGENE (2-methyl-4-methylene-6-phenyltetrahydro-2H-pyran); PEONILE (2-cyclohexylidene-2-phenylacetonitrile); PETALIA (2-cyclohexylidene-2-(o-tolyl)acetonitrile); PIVAROSE (2,2-dimethyl-2-pheylethyl propanoate); PRECYCLEMONE B (1-methyl-4-(4-methylpent-3-en-1-yl)cyclohex-3-enecarbaldehyde); PYRALONE (6-(sec-butyl)quinoline); RADJANOL SUPER ((E)-2-ethyl-4-(2,2,3-trimethylcyclopent-3-en-1-yl)but-2-en-1-ol); RASPBERRY KETONE (N112) (4-(4-hydroxyphenyl)butan-2-one); RHUBAFURANE (2,2,5-trimethyl-5-pentylcyclopentanone); ROSACETOL (2,2,2-trichloro-1-phenylethyl acetate); ROSALVA (dec-9-en-1-ol); ROSYFOLIA ((1-methyl-2-(5-methylhex-4-en-2-yl)cyclopropyl)-methanol); ROSYRANE SUPER (4-methylene-2-phenyltetrahydro-2H-pyran); SERENOLIDE (2-(1-(3,3-dimethylcyclohexyl)-ethoxy)-2-methylpropyl cyclopropanecarboxylate); SILVIAL (3-(4-isobutylphenyl)-2-methylpropanal); SPIROGALBANONE (1-(spiro[4.5]dec-6-en-7-yl)pent-4-en-1-one); STEMONE ((E)-5-methylheptan-3-one oxime); SUPER MUGUET ((E)-6-ethyl-3-methyloct-6-en-1-ol); SYLKOLIDE ((E)-2-((3,5-dimethylhex-3-en-2-yl)oxy)-2-methylpropyl cyclopropanecarboxylate); TERPINENE GAMMA (1-methyl-4-propan-2-ylcyclohexa-1,4-diene); TERPINOLENE (1-methyl-4-(propan-2-ylidene)cyclohex-1-ene); TERPINYL ACETATE (2-(4-methylcyclohex-3-en-1-yl)propan-2-yl acetate); TETRAHYDRO LINALOOL (3,7-dimethyloctan-3-ol); TETRAHYDRO MYRCENOL (2,6-dimethyloctan-2-ol); THIBETOLIDE (oxacyclohexadecan-2-one); TRIDECENE-2-NITRILE ((E)-tridec-2-enenitrile); UNDECAVERTOL ((E)-4-methyldec-3-en-5-ol); VELOUTONE (2,2,5-trimethyl-5-pentylcyclopentanone); VIRIDINE ((2,2-dimethoxyethyl)benzene); ZINARINE (2-(2,4-dimethylcyclohexyl)pyridine).

The core composition may also comprise at least one functional cosmetic ingredient. The functional cosmetic ingredients for use in the encapsulated composition are preferably hydrophobic.

Particularly useful functional cosmetic ingredients may be selected from the group consisting of emollients, smoothening ingredients, hydrating ingredients, soothing and relaxing ingredients, decorative ingredients, deodorants, anti-aging ingredients, cell rejuvenating ingredients, draining ingredients, remodeling ingredients, skin levelling ingredients, preservatives, anti-oxidants, antibacterial or bacteriostatic ingredients, cleansing ingredients, lubricating ingredients, structuring ingredients, hair conditioning ingredients, whitening ingredients, texturing ingredients, softening ingredients, anti-dandruff ingredients, and exfoliating ingredients.

Particularly useful functional cosmetic ingredients include, but are not limited to hydrophobic polymers, such as alkyldimethylsiloxanes, polymethylsil-sesquioxanes, polyethylene, polyisobutylene, styrene-ethylene-styrene and styrene-butylene-styrene block copolymers, and the like; mineral oils, such as hydrogenated isoparaffins, silicone oils and the like; vegetable oils, such as argan oil, jojoba oil, aloe vera oil, and the like; fatty acids and fatty alcohols and their esters; glycolipides; phospholipides; sphingolipides, such as ceramides; sterols and steroids; terpenes, sesquiterpenes, triterpenes and their derivatives; essential oils, such as Arnica oil, Artemisia oil, Bark tree oil, Birch leaf oil, Calendula oil, Cinnamon oil, Echinacea oil, Eucalyptus oil, Ginseng oil, Jujube oil, Helianthus oil, Jasmine oil, Lavender oil, Lotus seed oil, Perilla oil, Rosmary oil, Sandal wood oil, Tea tree oil, Thyme oil, Valerian oil, Wormwood oil, Ylang Ylang oil, Yucca oil and the like.

In particular, the functional cosmetic ingredient may be selected from the group consisting of Sandal wood oil, such as Fusanus Spicatus kernel oil; Panthenyl triacetate (CAS-No: 94089-18-6); Tocopheryl acetate; Tocopherol; Naringinin; Ethyl linoleate; Farnesyl acetate; Farnesol; Citronellyl methyl crotonate (CAS-No: 20770-40-5); Ceramide-2 (1-Stearoiyl-C18-Sphingosine, CAS-No: 100403-19-8); and mixtures thereof.

The process according to the present invention may also comprise the step of drying the slurry of obtained in step f), in order to obtain dry microcapsules.

Optionally, additional materials, such as carrier materials, such as salts, silicates, clays and carbohydrates, fire proofing materials, additional functional materials, such as fragrance ingredients, cosmetic ingredients, biologically active ingredients, and substrate enhancers, additional encapsulating materials, such as polysaccharides, proteins, alkoxysilanes, synthetic polymers and copolymers, surfactants and waxes.

Drying methods such as spray-drying, spray-coating, belt and drum drying may be employed. These methods are well known to the art.

In particular, the drying process may be accompanied by an additional encapsulation process, wherein an additional functional material is entrapped in an additional encapsulating material. For example, the slurry to be dried may comprise, additionally to the core-shell microcapsules obtained in the process according to the present invention, at least one non-encapsulated functional material and at least one water-soluble encapsulating material, so that the non-encapsulated functional material is entrapped in the water-soluble encapsulating material during drying. Typically, the at least one water-soluble encapsulating material comprises at least one hydrocolloid, such as starch octenyl succinate and gum acacia. The hydrocolloid promotes and stabilizes the dispersion of the non-encapsulated material in the aqueous phase of the slurry, so that, upon drying, a dry emulsion is formed around or coexisting with the core-shell microcapsules.

Combining at least two encapsulation processes has the advantage of providing different mechanisms for releasing the functional material, for example a combination of moisture-induced and mechanical stress-induced releases.

The drying step may also be accompanied or followed by mechanical or thermal treatment, such as spheronization, granulation and extrusion.

In a second aspect, the present invention provides an encapsulated composition obtainable by the process according to the present invention.

The encapsulated composition may be in the form of liquid slurries, powder, granulates, flakes or extrudates. The composition may be used as such, for example as fragrance booster, or in diluted form in a product.

Encapsulated compositions in the form of liquid slurries may comprise from 20 to 50 wt.-%, more particularly from 35 to 45 wt.-% of core-shell microcapsules.

Encapsulated compositions in solid form may comprise from 1 to 100 wt.-% of core-shell microcapsules. However, depending on the application or on the nature of the functional material, it may be preferable to limit or, in the contrary, to maximize the level of core-shell microcapsules in the solid form. For example, a limitation of the level of the core-shell microcapsules in the solid may be particularly desired if the encapsulated material is flammable, reactive, pungent or expensive.

Hence, the optimal level of encapsulated and/or entrapped fragrance ingredients in a solid form may be less than 50 wt.-%, more particularly less than 35 wt.-% and still more particularly less than 20 wt.-% or 15 wt.-%, depending on the flammability of such fragrance ingredients and the associated explosion risks.

In a third aspect, the present invention provides consumer product comprising an encapsulated composition as described herein above, wherein the consumer product is a fabric care product, a home care product or a personal care product.

The encapsulated compositions of the present invention that comprise fragrance ingredients may be used to perfume all manners of consumer products, including laundry care detergents, laundry care conditioners, fabric refreshers, personal care cleansing compositions, such as shampoos, bath and shower gels, liquid soaps, soap bars, personal care conditioning composition, such as hair care conditioners, bath and shower lotions, deodorant compositions, antiperspirant compositions, home care compositions, such as hard surface cleaners, heavy duty detergents.

Typical consumer products concerned by the present invention include personal care cleaning and cleansing compositions, such as shampoos, bath and shower gels, liquid soaps, soap bars, laundry care products, such as detergents, and home care products, such as hard surface cleaners.

The consumer products according to the present invention may be used for treating substrates, such as fabrics, skin, hair, animate and inanimate surfaces, hard surfaces, wherein the action of treating a substrate includes washing, cleansing, softening, caring, finishing, scenting and/or deodorizing this substrate.

In one aspect of the present invention, a consumer product contains the compositions as described herein above, preferably at a level of 0.005 to 5 wt.-%, more preferably from 0.01 to 1 wt.-% and still more preferably from 0.02 to 0.5 wt.-% of the consumer product.

In many cases, the consumer products concerned by the present invention contain surfactants, such as anionic, cationic, amphoteric or non-ionic surfactants.

The consumer products concerned by the present invention may contain acids or bases, or substances providing acidity or alkalinity, also referred to as acidity sources or alkalinity sources.

The consumer products concerned by the present invention may contain builders for reducing water hardness, such as phosphates, polyphosphates, polycarboxylates, sodium citrate, sodium carbonate, sodium silicate, sodium aluminosilicate (zeolite).

In many cases, the consumer products concerned by the present invention are liquid and may contain further additives, such as solvents, fillers, texturing agents, such as thickener and rheological aids, distributing aids, anti-redeposition agents, preservative agents, deodorizing agents, cosmetic ingredients, and surface enhancing agents.

The consumer product containing microcapsules of the present invention may contain at least one solvent selected from water-soluble solvents, or water-insoluble, or partially water-soluble solvents.

The consumer product containing microcapsules of the present invention may contain at least one texturing agent and/or colloid stabilizer, selected from rheology modifiers, thickener, gel-forming agents, thixotropic agents, and dispersing agents.

The consumer product containing microcapsules of the present invention may contain at least one silicone, selected from, but not limited to dimethicone, poly(dimethylsiloxabedimethylsiloxane), aminosilicone, such as amo-dimethiocone, trialkylammonium-silicone salts, ethoxylated silicones.

The consumer product containing microcapsules of the present invention may contain at least one cosmetic ingredient selected from, but not limited to emollients, moisturizing agents, anti-wrinkle agents, exfoliating agents, sunscreen agents, dyes, pigments, talcum, conditioning agents, hair styling agents, and antidandruff agents.

The consumer product containing microcapsules of the present invention may contain at least one fabric enhancing agent, selected from, but not limited to softening agents, optical brighteners and antistatic agents.

In a fourth aspect, the invention provides the use of the encapsulated composition as described herein above for obtaining a consumer product.

Further features and particular advantages of the present invention become apparent from the following examples.

Example 1: Preparation of Microcapsules

In Example 1.1, microcapsules according to the present invention were obtained by performing the steps of:

a) Preparing an aqueous phase by mixing 1.9 g of partially methylated poly-methylol-1,3,5-triamino-2,4,6-triazine precondensate (Luracoll SD, ex BASF, 70 wt.-% active content), 2.5 g of poly(methacrylamido-propyltrimethylammonium chloride-co-acrylic acid) cationic copolymer, having 71 mol-% methacrylamidopropyltrimethylammonium chloride and and 29 mol-% acrylic acid, prepared according to WO 2016/207180 A1 (Example 1), and 44 g of water;

b) Adding 35.5 g of perfume (ex Givaudan) in the aqueous phase under stirring;

c) Adding 0.7 g of trifunctional araliphatic isocyanate (Quix 175, ex Covestro, 75.8 wt.-% active content in ethyl acetate) to the mixture obtained in step b);

d) Emulsifying the mixture obtained in step c) at a stirring speed of 950 rpm;

e) Adding about 25 g of a 10 vol.-% formic acid in water to lower the pH of the emulsion to a value of 3.5 $\pm$ 0.3;

f) Heating the emulsion to a temperature of 85 $\pm$ 5 °C for about 2 h;

g) Letting the slurry cool to room temperature and adding about 5 g of a mixture of 20 vol.-% ammonia in water, in order to obtain a slurry of microcapsules having a pH of 5.5 $\pm$ 0.5.

The solid content of the slurry was measured by using a thermo-balance operating at 120 °C. The solid content, expressed as weight percentage of the initial slurry deposited on the balance, was taken at the point where the drying-induced rate of weight change had dropped below 0.1 %/min. The ratio of the measured solid content to the theoretical solid content calculated based on the weight of perfume and encapsulating materials involved is taken as a measurement of encapsulation yield, expressed in wt.-%.

The slurry was free of agglomerate, the solid content of the slurry obtained was 43 wt.-%, the volume average size (D50) of the capsules was 10 $\mu$m, the zeta-potential was +14 mV at pH 4 and +20 mV at pH 7 and the encapsulation efficiency was 100 %.

In comparative Example 1.2, microcapsules were obtained by applying the procedure disclosed in WO 2016/207180 A1, Example 1, by performing the steps of:

a) Preparing an aqueous phase by mixing 1.9 g of partially methylated poly-methylol-1,3,5-triamino-2,4,6-triazine precondensate (Luracoll SD, ex BASF, 70 wt.-% active content), 0.67 g of resorcinol, and 2.5 g of poly(methacrylamidopropyltrimethylammonium chloride-co-acrylic acid) cationic copolymer, having 71 mol-% methacrylamidopropyl-trimethylammonium chloride, 29 mol-% acrylic acid, prepared according to WO 2016/207180 A1 (Example 1), and 44 g of water;

b) Adding 35.5 g of perfume (ex Givaudan) in the aqueous phase under stirring;

c) Emulsifying the mixture obtained in step c) at a stirring speed of 950 rpm;

d) Adding about 25 g of a 10 vol.-% formic acid in water to lower the pH of the emulsion to a value of 3.5 $\pm$ 0.3;

e) Heating the emulsion to a temperature of 85 $\pm$ 5 °C for 1 h;

f) Letting the slurry cool to room temperature and adding about 5 g of a mixture of 20 vol.-% ammonia in water, in order to obtain a slurry of microcapsules having a pH of 6.2 $\pm$ 0.5.

The resulting capsule slurry was free from agglomerates, the solid content of the slurry was 42.8 wt.-%, the volume average size (D50) of the microcapsules was 7.5 $\mu$m, the zeta-potential was +29 mV at pH 4 and +22 mV at pH 7 and the encapsulation efficiency was 100 %.

In comparative Example 1.3, microcapsules were obtained by using the same process as in Example 1.1, but an anionic copolymer of ethylene and maleic anhydride was used instead of the cationic copolymer, and the trifunctional araliphatic isocyanate was dissolved in the perfume before emulsification. This was achieved by performing the steps of:

a) Preparing an aqueous phase by mixing 1.9 g of partially methylated poly-methylol-1,3,5-triamino-2,4,6-triazine precondensate (Luracoll SD, ex BASF, 70 wt.-% active content), 0.8 g of poly(maleic anhydride-co-ethylene) (ZeMac E60) and 47 g of water;

b) Preparing a core phase by mixing 35.5 g of perfume (ex Givaudan) and 0.7 g of trifunctional araliphatic isocyanate (Takenate D-100N, ex Mitsui)

c) Adding the core phase to the aqueous phase;

d) Emulsifying the mixture obtained in step c) at a stirring speed of 950 rpm;

e) Reducing the stirring speed to 625 $\pm$ 25 rpm and adding about 25 g of a 10 vol.-% formic acid in water to lower the pH of the emulsion to a value of 3.5 $\pm$ 0.3;

f) Heating the emulsion to a temperature of 90 °C for 1 h;

g) Letting the slurry cool to room temperature and adding about 5 g of a mixture of 20 vol.-% ammonia in water, in order to obtain a slurry of microcapsules having a pH of 6.2 $\pm$ 0.5.

The slurry was free from agglomerates, the solid content of the slurry obtained in Example 1.3 was 39.3 wt.-%, the volume average size (D50) of the microcapsules was 10 $\mu$m, the zeta-potential was -49 mV at pH 4 and -52 mV at pH 7, and the encapsulation efficiency was 100 %.

Example 2: Measurement of Perfume Leakage in Model Extractive Medium

The model extractive medium was a system consisting of an aqueous solution of ethanol at an initial concentration of 30 vol.-% co-existing with an immiscible cyclohexane phase.

In a first step, 10 ml of cyclohexane are put into a vial. Second, 1.8 ml of a 30 vol.-% ethanol in water is added to the vial. After equilibration, taking into account the partition coefficient of ethanol between cyclohexane and the water of 0.03 (see A.W. Islam, A. Zavvadi, V.N. Kabadi, Chem. Process Eng. 2012, 33, 243-253), the percentage of ethanol in the aqueous phase is 25 vol.-% and the percentage of ethanol referred to the whole system is 4.6 vol.-%.

In a second step, the slurry to be assessed is diluted in such a way that the perfume concentration in the diluted slurry is 10 wt.-% and 200 $\mu$L of this diluted slurry is added to the vial.

In a third step, the vial is submitted to horizontal mixing on an elliptic xy-mixing equipment operating at a 250 rpm for 4 hours (shaking in the z direction is avoided).

In a fourth step, the upper cyclohexane phase containing the extracted perfume is analyzed spectrophotometrically by using a UV/visible light spectrometer. The perfume concentration is determined by measuring the intensity of the absorbed UV/visible light at the maximum absorbance wavelength, which has been determined previously by using a reference perfume / cyclohexane solution of known concentration. This latter reference solution is used as an external standard for the quantification of the extracted perfume. The leakage value is defined as the percentage of the encapsulated perfume that has been recovered in the hexane phase.

Representative leakage values are given in Table 1 hereunder.

Table 1: Perfume leakage in model extractive medium

| Sample | Leakage in wt.-% of the initial amount of encapsulated perfume |
|---|---|
| SAMPLE 1.1 | 10 |
| SAMPLE 1.2 | 20 |
| SAMPLE 1.3 | 59 |

As apparent from these results, the microcapsules obtained by applying the process according to the present invention show a lower leakage compared to the one obtained with resorcinol as cross-linker (Example 1.2). The results show also that combining a cationic stabilizing polymer and the addition of trifunctional araliphatic isocyanate in the emulsion leads to microcapsules that show significantly less perfume leakage, compared to microcapsules obtained by using an anionic stabilizing polymer with the isocyanate added to the perfume phase (Example 1.3).

Example 3: Olfactive Performance in Fabric Care Conditioner

The slurries of core-shell microcapsules obtained in Example 1.1 and 1.2 were incorporated into a model fabric care conditioner having the composition shown in Table 2. The slurry obtained in Example 1.3 was not tested due to excessive leakage. In the tests, the level of encapsulated perfume was 0.5 wt.-% based on the total weight of the conditioner. The pH of the conditioner was 3.

Table 2: Conditioner composition

| Ingredient | Supplier | INCI name | Quantity [wt.-%] |
|---|---|---|---|
| Calcium Chloride | VWR | Calcium chloride | 0.5 |
| Stepantex SP-90 | Stepan | Dialkylester Ammonium Methosulfate | 11.1 |
| Eumulgin CO-40 | BASF | PEG-40 Hydrogenated Castor Oil | 1 |
| Sodium benzoate | VWR | Sodium benzoate | 0.3 |
| Citric acid | VWR | Citric acid | q.s. pH 3 |
| Water | | | q.s. 97 |
| Fragrance | | | 2 |
| Encapsulated composition (slurry) | | | 0.5 |

Terry towels were submitted to a rinse cycle in a front-loaded wash machine. The amount of conditioner was 35 g for a towel load of 1 kg and the total volume of water was 20 L.

Olfactive evaluations were performed using both freshly prepared conditioner and after aging the conditioners for one month at 37 °C.

The pre-rub olfactive evaluation was performed on wet laundry directly out of the machine and after 4 h. For this evaluation, the terry toweling was handled carefully in order to minimize the risk of breaking the microcapsules mechanically. The post-rub olfactive evaluation was performed after line drying the terry toweling for 24 hours at room temperature. This evaluation was performed by gently rubbing one part of the terry toweling on another part of same terry toweling. The olfactive performance (intensity) has been assessed by a panel of 4 experts rated on a scale of 1-5 (1 = barely noticeable, 2 = weak, 3 = medium, 4 = strong and 5 = very strong). In such evaluation, the post-rub score measures the additional impact induced by microcapsule breakage, compared to the pre-rub intensity. The results are reported on Table 3.

Table 3: Olfactive performance of encapsulated compositions 1.1 and 1.2 in laundry care conditioner

| Sample | Pre-rub intensity (t=0) | Post-rub intensity (t=0) | Pre-rub intensity (t=1 month @ 37 °C) | Post-rub intensity (t=1 month @ 37 °C) |
|---|---|---|---|---|
| 1.1 | 2.0 | 2.3 | 2.3 | 2.2 |
| 1.2 | 2.2 | 2.0 | 1.8 | 2.0 |

As apparent form the results of Table 3, the encapsulated composition according to the present invention is at least as good as the benchmark for the freshly prepared conditioner and perform significantly better than the benchmark after the conditioner has undergone 1 month storage at 37 °C.

Example 4: Measurement of Color

The difference between the coloration of microcapsule slurries of Example 1.1 and Example 1.2 with respect to calibrated white plate was measured by using a Chroma Meter CR-400/410 colorimeter. This instrument provides the a*, b*, and L* value of the so-called CIE 1976 L*a*b* color space of the Commission of Illumination, according to norm ISO/CIE 11664-4:2019(E), and the ΔE value, which describes the difference between two colors in the CIE 1976 L*a*b* color space and is given by Equation 1:

$$\Delta E = (L_1^* - L_2^*)^2 + (a_1^* - a_2^*)^2 + (b_1^* - b_2^*)^2 \qquad \text{Equation 1}$$

The measurement was made on freshly prepared slurries and on slurries submitted to accelerated aging, combining 3 weeks storage at 50 °C and 3 days exposure to artificial sun light.

The results are reported in Table 4.

Table 4: Coloration values according to CIE 1976 L*a*b* color space of the Commission of Illumination

| Example | L* | a* | b* | ΔE |
|---|---|---|---|---|
| 1.1 (t=0) | 83.72 | -0.61 | 0.11 | 1.06 |
| 1.1 (aged) | 82.40 | -0.99 | -1.01 | 1.6 |
| 1.2 (t=0) | 77.92 | 3.03 | 9.17 | 10.84 |
| 1.2 (aged) | 73.89 | 5.12 | 10.02 | 14.55 |

These results show that the color of the microcapsule slurries obtained by the process of the invention is close to the white standard and that this whiteness is kept over aging, whereas the color of the reference microcapsule slurries of the comparative example is already different from the white standard at time zero and that the coloration of these latter slurries increases over aging.

**Claims**

1. A process for obtaining an encapsulated composition comprising at least one cationic core-shell microcapsule, wherein the at least one cationic core-shell microcapsule comprises a core comprising at least one functional material and a shell surrounding the core, wherein the shell comprises a resin formed by reaction of at least one trifunctional araliphatic isocyanate with at least one partially alkylated amino-aldehyde pre-condensate, the process comprising the steps of:

   a) Providing a core composition;
   b) Providing an aqueous phase comprising the at least one partially alkylated amino-aldehyde pre-condensate and a cationic stabilizing polymer;
   c) Emulsifying the core composition provided in step a) with the aqueous phase provided in step b) in order to obtain an emulsion of core composition droplets having a volume average size of 1 to 100 μm, preferably 5 to 50 μm, even more preferably 7 to 20 μm;
   d) Heating the emulsion obtained in step c) to a temperature of from 70 to 95 °C, preferably from 80 to 90 °C; preferably at a pH of 2.0 to 4.5, more preferably of 2.5 to 4.0, still more preferably of 3.0 to 3.8, even more preferably of 3.4 to 3.6, in order to obtain core-shell microcapsules;

e) Optionally: Adding a formaldehyde scavenging agent; and

f) Letting the mixture obtained in step d) or step e) cool to room temperature, in order to obtain a slurry of microcapsules;

wherein the at least one trifunctional araliphatic isocyanate is added to the aqueous phase or the emulsion before, during or after step c).

2. The process according to claim 1, wherein the at least one trifunctional araliphatic isocyanate is an adduct of an aliphatic triol with three araliphatic diisocyanates; optionally an adduct of 2-ethylpropane-1,2,3-triol or 2-ethyl-2-(hydroxymethyl)propane-1,3-diol with a compound selected from the group consisting of 1-isocyanato-2-(isocyanatomethyl)-benzene, 1-isocyanato-3-(isocyanatomethyl)benzene and 1-isocyanato-4-(isocyanatomethyl)benzene; optionally an adduct of 2-ethylpropane-1,2,3-triol with 1-isocyanato-3-(isocyanatomethyl)benzene.

3. The process according to one of claims 1 or 2, wherein the at least one partially alkylated amino-aldehyde precondensate is selected from the group consisting of partially methylated polymethylol-1,3,5-triamino-2,4,6-triazine pre-condensates, partially methylated polymethylolurea, partially methylated polymethylol-benzoguanamine and partially methylated polymethylol-glycouril, more preferably partially methylated polymethylol-1,3,5-triamino-2,4,6-triazine pre-condensates.

4. The process according to claim 3, wherein the partially methylated polymethylol-1,3,5-triamino-2,4,6-triazine pre-condensate is obtainable by reacting melamine, formaldehyde and methanol, and wherein the molar ratio of melamine to formaldehyde is from 1:3 to 1:6 and the molar ratio of melamine to alcohol is 1:2 to 1:4.

5. The process according to one of claims 1 to 4, wherein the weight ratio of the at least one trifunctional araliphatic isocyanate to the at least one partially alkylated amino-aldehyde pre-condensate is 0.25 to 0.50, preferably from 0.3 to 0.4.

6. The process according to one of claims 1 to 5, wherein the cationic stabilizing polymer is a copolymer obtainable by copolymerizing methacrylamidopropyltrimethylammonium chloride, acrylic acid or methacrylic acid and, optionally, acrylamide.

7. The process according to claim 6, wherein the cationic stabilizing copolymer is a copolymer of 2 to 99 mol %, more particularly 30 to 95 mol %, still more particularly 60 to 90 mol %, of methacrylamidopropyltrimethylammonium chloride (MAPTAC); and 1 to 98 mol %, more particularly 5 to 70 mol %, still more particularly 10 to 40 mol %, of acrylic acid, and 0 to 50 mol %, more particularly 0.1 to 5 mol %, of acrylamide.

8. The process according to one of claims 1 to 7, additionally comprising the step of adding, between step d) and step f), preferably between step e) and step f), a suspending agent.

9. The process according to claim 8, wherein the suspending agent is a cationic polymer, preferably a cationic copolymer.

10. The process according to one of claims 1 to 9, additionally comprising the step of adding at least one preservative to the mixture obtained in step d) or to the slurry of microcapsules obtained in step f).

11. The process according to one of claims 1 to 10, wherein the core composition comprises at least one functional material selected from group consisting of fragrance ingredients, cosmetic ingredients, biologically active ingredients, and substrate enhancers.

12. The process according to claim 11, wherein the core composition comprises at least one fragrance ingredient selected from the group consisting of 2,6,10-trimethylundec-9-enal; 2-(tert-butyl)cyclohexyl acetate; decanal; 2-methyldecanal; undec-10-enal); undecanal; dodecanal; 2-methylundecanal; (E)-undec-9-enal; (E)-dodec-2-enal; allyl 2-(isopentyloxy)acetate; allyl 3-cyclohexylpropanoate; allyl heptanoate; 1-((2-(tert-butyl)cyclohexyl)oxy)-butan-2-ol; 1,3,4,5,6,7-hexahydro-.beta.,1,1,5,5-pentamethyl-2H-2,4a-methanonaphthalene-8-ethanol; pentyl 2-hydroxybenzoate; 1-(3,3-dimethylcyclohexyl)ethyl formate; (1R,2S,4R)-2'-isopropyl-1,7,7-trimethyl-spiro[bicyclo[2.2.1]heptane-2,4'-[1,3]dioxane]; 8-(sec-butyl)-5,6,7,8-tetra-hydroquinoline); (ethoxymethoxy)-cyclododecane; (1S,2R,5R)-2-ethoxy-2,6,6-trimethyl-9-methylene-bicyclo[3.3.1]nonane; (2S,4S)-1,7,7-trimethyl-bicyclo[2.2.1]-heptan-2-yl acetate; 1-butoxy-1-oxopropan-2-yl butyrate; 4-(tert-butyl)cyclohexyl acetate; (Z)-4,11,11-trimethyl-8-methylene-bicyclo[7.2.0]-undec-4-ene; 1,1,2,3,3-pentamethyl-2,3,6,7-tetrahydro-1H-inden-4(5H)-one; 5-tert-butyl-2-methyl-5-propyl-2H-furan; (E)-3,7-dimethylocta-2,6-dienal; (E)-3,7-dimethylocta-2,6-dienal; (Z)-1,1-diethoxy-3,7-dimethylocta-2,6-diene; 3,7-dimethyloct-6-enal; 3,7-dimethyloct-6-en-1-ol 3,7-dimethyloct-6-en-1-yl acetate; 3,7-dimethyloct-6-en-1-yl formate; 3,7-dimethyloct-6-enenitrile; 3,7-dimethyloct-6-en-1-yl propionate; dodecanenitrile; 4-cyclohexyl-2-methylbutan-2-ol; (Z)-3-methylcyclotetradec-5-enone; 3-(4-isopropylphenyl)-2-methylpropanal; (allyl 2-(cyclohexyloxy)acetate; cyclohexyl 2-hydroxybenzoate; 8,8-dimethyl-1,2,3,4,5,6,7,8-octahydronaphthalene-2-carbaldehyde; (E)-1-(2,6,6-trimethylcyclohexa-1,3-dien-1-yl)but-2-en-1-one; (E)-1-(2,6,6-trimethylcyclohex-2-en-1-yl)but-2-en-1-one; (E)-1-(2,6,6-trimethyl-cyclohex-3-en-1-yl)but-2-en-1-one; (E)-dec-4-enal; 2-pentylcyclopentanone; propanedioic acid 1-(1-(3,3-dimethylcyclohexyl)ethyl) 3-ethyl ester; 3-methyl-2-pentylcyclopent-2-enone; 2-methyl-1-phenylpropan-2-ol; 2-methyl-1-phenylpropan-2-yl acetate; 2-methyl-1-phenylpropan-2-yl butyrate; 4,7-dimethyloct-6-en-3-one; 2,6-dimethylheptan-2-ol; 1-methyl-4-(prop-1-en-2-yl)cyclohex-1-ene; (E)-4-((3aS,7aS)-hexahydro-1H-4,7-methanoinden-5(6H)-ylidene)butanal; (E)-3-methyl-5-(2,2,3-trimethylcyclopent-3-en-1-yl)pent-4-en-2-ol; ethyl hexanoate; ethyl octanoate; (E)-3,7-dimethylnona-1,6-dien-3-ol; (Z)-3,7-dimethylnona-1,6-dien-3-yl acetate; ethyl heptanoate; ethyl 2,6,6-trimethylcyclohexa-1,3-diene-1-carboxylate; (1s,4s)-1,3,3-trimethyl-2-oxa-bicyclo[2.2.2] octane; (2S)-1,3,3-trimethylbicyclo[2.2.1] heptan-2-yl acetate; (1S,2R,4R)-1,3,3-trimethylbicyclo[2.2.1]heptan-2-o1); 1-(3,5,5,6,8,8-hexa-methyl-5,6,7,8-tetrahydronaphthalen-2-yl)ethanone; 3-(4-ethylphenyl)-2,2-dimethylpropanal; 3-(3-isopropylphenyl)butanal; (3aR,6S,7aS)-3a,4,5,6,7,7a-hexahydro-1H-4,7-methanoinden-6-yl propionate; 2,4,6-trimethyl-4-phenyl-1,3-dioxane; 2-(sec-butyl)cyclohexanone); (3aS,4S, 7R, 7aS) -ethyl octahydro-1H-4,7-methanoindene-3a-carboxylate; 2-methyldecanenitrile; 1-(3,3-dimethylcyclohex-1-en-1-yl)pent-4-en-1-one; (3aR,6S,7aS)-3a,4,5,6,7,7a-hexahydro-1H-4,7-methanoinden-6-yl isobutyrate; (E)-3,7-dimethylocta-2,6-dien-1-ol; (E)-3,7-dimethylocta-2,6-dien-1-yl acetate; (E)-3,7-dimethyloctа-2,6-2-one; 1-(2,3,8,8-tetramethyl-1,2,3,4,5,6,7,8-octahydronaphthalen-2-yl)ethanone); 2,4,6-trimethylcyclohex-3-enecarbaldehyde; 3,5,5-dien-1-yl isobutyrate; ethyl 2-ethyl-6,6-dimethylcyclohex-2-enecarboxylate; (E)-oxacyclohexadec-12-en-2-one; methyl 3-oxo-2-pentylcyclopentaneacetate; (2S)-ethyl 3-isopropylbicyclo[2.2.1] hept-5-ene-2-carboxylate; (Z)-hex-3-en-1-yl butyrate; (E)-2-benzylideneoctanal; hexyl isobutyrate; hexyl 2-hydroxybenzoate; 4,4a,5,9b-tetrahydroindeno[1,2-d][1,3]dioxine; (E)-4-(2,6,6-trimethylcyclohex-1-en-1-yl)but-3-en-2-one; (E)-4-(2,6,6-trimethylcyclohex-2-en-1-yl)but-3-en-2-one; (E)-4-(2,5,6,6-tetramethylcyclohex-2-en-1-yl)but-3-en-trimethylhexyl acetate; isopropyl 2-methyl butanoate; (E)-3-methyl-4-(2,6,6-trimethylcyclohex-2-en-1-yl)but-3-en-2-one; (3aR,6S,7aS)-3a,4,5,6,7,7a-hexahydro-1H-4,7-methanoinden-6-yl acetate; (Z)-3-methyl-2-(pent-2-en-1-yl)cyclopent-2-enone; (Z)-3,4,5,6,6-pentamethylhept-3-en-2-one; (Z)-1-(1-ethoxyethoxy)hex-3-ene (2E,6Z)-3,7-dimethylnona-2,6-dienenitrile; (Z)-hex-3-en-1-yl methyl carbonate; 3-(4-(tert-butyl)phenyl)-2-methylpropanal; 3,7-dimethylocta-1,6-dien-3-ol; 3,7-dimethylocta-1,6-dien-3-yl acetate; (4E)-9-hydroxy-5,9-dimethyl-4-decenal; 2-methyl-4-oxo-4H-pyran-3-yl isobutyrate; ethyl 2-methylpentanoate; 2,6-dimethylhept-5-enal; 2-isopropyl-5-methylcyclohexanol; 2-isopropyl-5-methylcyclohexanone; 1-((1S,8aS)-1,4,4,6-tetramethyl-2,3,3a,4,5,8-hexahydro-1H-5,8a-methanoazulen-7-yl)ethanone; undecan-2-one; methyl non-2-ynoate; 6,6-dimethoxy-2,5,5-trimethylhex-2-ene; 4-(4-methylpent-3-en-1-yl)cyclohex-3-enecarbaldehyde; 2-(2-(4-methylcyclohex-3-en-1-yl)propyl)cyclopentanone; 2-methyl-6-methyleneoct-7-en-2-yl acetate; (E)-methyl non-2-enoate; (Z)-3,7,11-trimethyldodeca-1,6,10-trien-3-yl acetate; (Z)-3,7-dimethylocta-2,6-dien-1-yl acetate; 6,8-dimethylnonan-2-ol; (Z)-non-6-enal; 3-(4-isobutyl-2-methylphenyl)propanal; 4-(tert-pentyl)cyclohexanone; 2-ethyl-N-methyl-N-(m-tolyl)butanamide; 2-methyl-4-methylene-6-phenyltetrahydro-2H-pyran; 2-cyclohexylidene-2-phenylacetonitrile; 2-cyclohexylidene-2-(o-tolyl)acetonitrile; 2,2-dimethyl-2-pheylethyl propanoate; 1-methyl-4-(4-methylpent-3-en-1-yl)cyclohex-3-enecarbaldehyde; 6-(sec-butyl)quinoline; (E)-2-ethyl-4-(2,2,3-trimethylcyclopent-3-en-1-yl)but-2-en-1-ol; 4-(4-hydroxyphenyl)butan-2-one; 2,2,5-trimethyl-5-pentylcyclopentanone; 2,2,2-trichloro-1-phenylethyl acetate); ROSALVA (dec-9-en-1-ol; (1-methyl-2-(5-methylhex-4-en-2-yl)cyclopropyl)-methanol; 4-methylene-2-phenyltetrahydro-2H-pyran; 2-(1-(3,3-dimethylcyclohexyl)-ethoxy)-2-methylpropyl cyclopropanecarboxylate; 3-(4-isobutylphenyl)-2-methylpropanal; 1-(spiro[4.5]dec-6-en-7-yl)pent-4-en-1-one; (E)-5-methylheptan-3-one oxime; (E)-6-ethyl-3-methyloct-6-en-1-ol; (E)-2-((3,5-dimethylhex-3-en-2-yl)oxy)-2-methylpropyl cyclopropanecarboxylate; 1-methyl-4-propan-2-ylcyclohexa-1,4-diene; 1-methyl-4-(propan-2-ylidene)cyclohex-1-ene; 2-(4-methylcyclohex-3-en-1-yl)propan-2-yl acetate; 3,7-dimethyloctan-3-ol; 2,6-dimethyloctan-2-ol; oxacyclohexadecan-2-one; (E)-tridec-2-enenitrile; (E)-4-methyldec-3-en-5-ol; 2,2,5-trimethyl-5-pentylcyclopentanone; (2,2-dimethoxyethyl)benzene and 2-(2,4-dimethylcyclohexyl)pyridine).

13. An encapsulated composition obtainable by the process according to one of claims 1 to 12.

14. A consumer product comprising the encapsulated composition according to claim 13, wherein the consumer product is a fabric care product, a home care product or a personal care product.

15. Use of the encapsulated composition according to claim 13 for obtaining a consumer product.


**Patentansprüche**

1. Verfahren zum Erhalten einer verkapselten Zusammensetzung, die mindestens eine kationische Kern-Schale-Mikrokapsel umfasst, wobei die mindestens eine kationische Kern-Schale-Mikrokapsel einen Kern, der mindestens ein funktionelles Material umfasst, und eine den Kern umgebende Schale umfasst, wobei die Schale ein Harz umfasst, das durch Umsetzung mindestens eines trifunktionellen araliphatischen Isocyanats mit mindestens einem teilalkylierten Aminoaldehyd-Vorkondensat gebildet wird, wobei das Verfahren die Schritte umfasst:

   a) Bereitstellen einer Kernzusammensetzung;
   b) Bereitstellen einer wässrigen Phase, die das mindestens eine teilalkylierte Aminoaldehyd-Vorkondensat und ein kationisches stabilisierendes Polymer umfasst;
   c) Emulgieren der in Schritt a) bereitgestellten Kernzusammensetzung mit der in Schritt b) bereitgestellten wässrigen Phase, um eine Emulsion von Kernzusammensetzungströpfchen mit einer volumenmittleren Größe von 1 bis 100 $\mu$m, vorzugsweise 5 bis 50 $\mu$m, noch weiter bevorzugt 7 bis 20 $\mu$m, zu erhalten;
   d) Erhitzen der in Schritt c) erhaltenen Emulsion auf eine Temperatur von 70 bis 95 °C, vorzugsweise von 80 bis 90 °C; vorzugsweise bei einem pH-Wert von 2,0 bis 4,5, weiter bevorzugt von 2,5 bis 4,0, noch weiter bevorzugt von 3,0 bis 3,8, noch weiter bevorzugt von 3,4 bis 3,6, um Kern-Schale-Mikrokapseln zu erhalten;
   e) gegebenenfalls: Zugeben eines Formaldehydfängers und
   f) Abkühlen der in Schritt d) oder Schritt e) erhaltenen Mischung auf Raumtemperatur, um eine Aufschlämmung von Mikrokapseln zu erhalten;

   wobei das mindestens eine trifunktionelle araliphatische Isocyanat vor, während oder nach Schritt c) zu der wässrigen Phase oder der Emulsion gegeben wird.

2. Verfahren nach Anspruch 1, wobei es sich bei dem mindestens einen trifunktionellen araliphatischen Isocyanat um ein Addukt eines aliphatischen Triols mit drei araliphatischen Diisocyanaten, gegebenenfalls ein Addukt von 2-Ethylpropan-1,2,3-triol oder 2-Ethyl-2-(hydroxymethyl)propan-1,3-diol mit einer Verbindung, die aus der Gruppe bestehend aus 1-Isocyanat-2-(Isocyanatmethyl)benzol, 1-Isocyanat-3-(Isocyanatmethyl)benzol und 1-Isocyanat-4-(Isocyanatmethyl)benzol ausgewählt ist, gegebenenfalls ein Addukt von 2-Ethylpropan-1,2,3-triol mit 1-Isocyanat-3-(Isocyanatmethyl)benzol, handelt.

3. Verfahren nach Anspruch 1 oder 2, wobei das mindestens eine teilalkylierte Aminoaldehyd-Vorkondensat aus der Gruppe bestehend aus teilmethylierten Polymethylol-1,3,5-triamin-2,4,6-triazin-Vorkondensaten, teilmethyliertem Polymethylolharnstoff, teilmethyliertem Polymethylolbenzoguanamin und teilmethyliertem Polymethylolglykouril, weiter bevorzugt teilmethylierten Polymethylol-1,3,5-triamin-2,4,6-triazin-Vorkondensaten, ausgewählt ist.

4. Verfahren nach Anspruch 3, wobei das teilmethylierte Polymethylol-1,3,5-triamin-2,4,6-triazin-Vorkondensat durch Umsetzung von Melamin, Formaldehyd und Methanol erhältlich ist und wobei das Molverhältnis von Melamin zu Formaldehyd von 1:3 bis 1:6 beträgt und das Molverhältnis von Melamin zu Alkohol 1:2 bis 1:4 beträgt.

5. Verfahren nach einem der Ansprüche 1 bis 4, wobei das Gewichtsverhältnis des mindestens einen trifunktionellen araliphatischen Isocyanats zu dem mindestens einen teilalkylierten Aminoaldehyd-Vorkondensat 0,25 bis 0,50, vorzugsweise 0,3 bis 0,4, beträgt.

6. Verfahren nach einem der Ansprüche 1 bis 5, wobei es sich bei dem kationischen stabilisierenden Polymer um ein Copolymer handelt, das durch Copolymerisieren von Methacrylamidopropyltrimethylammoniumchlorid, Acrylsäure oder Methacrylsäure und gegebenenfalls Acrylamid erhältlich ist.

7. Verfahren nach Anspruch 6, wobei es sich bei dem kationischen stabilisierenden Copolymer um ein Copolymer von 2 bis 99 Mol-%, spezieller 30 bis 95 Mol-%, noch spezieller 60 bis 90 Mol-%, Methacrylamidopropyltrimethylammoniumchlorid (MAPTAC) und 1 bis 98 Mol-%, spezieller 5 bis 70 Mol-%, noch spezieller 10 bis 40 Mol-% Acrylsäure und 0 bis 50 Mol-%, spezieller 0,1 bis 5 Mol-%, Acrylamid handelt.

8. Verfahren nach einem der Ansprüche 1 bis 7, zusätzlich umfassend den Schritt des Zugebens eines Suspendiermittels zwischen Schritt d) und Schritt f), vorzugsweise zwischen Schritt e) und Schritt f).

9. Verfahren nach Anspruch 8, wobei es sich bei dem Suspendiermittel um ein kationisches Polymer, vorzugsweise ein kationisches Copolymer, handelt.

10. Verfahren nach einem der Ansprüche 1 bis 9, zusätzlich umfassend den Schritt des Zugebens mindestens eines Konservierungsmittels zu der in Schritt d) erhaltenen Mischung oder zu der in Schritt f) erhaltenen Aufschlämmung von Mikrokapseln.

11. Verfahren nach einem der Ansprüche 1 bis 10, wobei die Kernzusammensetzung mindestens ein funktionelles Material umfasst, das aus der Gruppe bestehend aus Duftstoffbestandteilen, kosmetischen Bestandteilen, biologisch aktiven Bestandteilen und Substratverstärkern ausgewählt ist.

12. Verfahren nach Anspruch 11, wobei die Kernzusammensetzung mindestens einen Duftstoffbestandteil umfasst, der aus der Gruppe bestehend aus 2,6,10-Trimethylundec-9-enal; 2-(tert-butyl)cyclohexylacetat; Decanal; 2-Methyldecanal; Undec-10-enal); Undecanal; Dodecanal; 2-Methylundecanal; (E)-Undec-9-enal; (E)-Dodec-2-enal; Allyl-2-(isopentyloxy)acetat; Allyl-3-cyclohexylpropanoat; Allylheptanoat; 1-((2-(tert-Butyl)cyclohexyl)oxy)-butan-2-ol; 1,3,4,5,6,7-Hexahydro-.beta.,1,1,5,5-pentamethyl-2H-2,4a-methanonaphthalin-8-ethanol; Pentyl-2-Hydroxybenzoat; 1-(3,3-dimethylcyclohexyl)ethylformiat; (1R,2S,4R)-2'-Isopropyl-1,7,7-trimethyl-spiro[bicyclo[2.2.1]heptan-2,4'-[1,3]dioxan]; 8-(sec-Butyl)-5,6,7,8-tetra-hydrochinolin); (Ethoxymethoxy)-cyclododecan; (1S,2R,5R)-2-Ethoxy-2,6,6-trimethyl-9-methylenbicyclo[3.3.1]nonan; (2S,4S)-1,7,7-trimethylbicyclo[2.2.1]-heptan-2-yl-acetat; 1-Butoxy-1-oxopropan-2-butyrat; 4-(tert-Butyl)cyclohexylacetat; (Z)-4,11,11-Trimethyl-8-methylen-bicyclo[7.2.0]-undec-4-en; 1,1,2,3,3-Pentamethyl-2,3,6,7-tetrahydro-1H-inden-4(5H)-on; 5-tert-Butyl-2-methyl-5-propyl-2H-furan; (E)-3,7-Dimethylocta-2,6-dienal; (E)-3,7-Dimethylocta-2,6-dienal; (Z)-1,1-Diethoxy-3,7-dimethylocta-2,6-dien; 3,7-Dimethyloct-6-enal; 3,7-Dimethyloct-6-en-1-ol; 3,7-dimethyloct-6-en-1-yl-acetat; 3,7-dimethyloct-6-en-1-yl-formiat; 3,7-Dimethyloct-6-ennitril; 3,7-Dimethyloct-6-en-1-yl-propionat; Dodecannitril; 4-Cyclohexyl-2-methylbutan-2-ol; (Z)-3-Methylcyclotetradec-5-enon; 3-(4-Isopropylphenyl)-2-methylpropanal; (Allyl-2-(Cyclohexyloxy)acetat; Cyclohexyl-2-Hydroxybenzoat; 8,8-Dimethyl-1,2,3,4,5,6,7,8-octahydronaphthalin-2-carbaldehyd; (E)-1-(2,6,6-Trimethylcyclohexa-1,3-dien-1-yl)but-2-en-1-on; (E)-1-(2,6,6-Trimethylcyclohex-2-en-1-yl)but-2-en-1-on; (E)-1-(2,6,6-Trimethyl-cyclohex-3-en-1-yl)but-2-en-1-on; (E)-Dec-4-enal; 2-Pentylcyclopentanon; Propanedisäure-1-(1-(3,3-dimethylcyclohexyl)ethyl)-3-ethylester; 3-Methyl-2-pentylcyclopent-2-enon; 2-Methyl-1-phenylpropan-2-ol; 2-Methyl-1-phenylpropan-2-yl-acetat; 2-Methyl-1-phenylpropan-2-yl-butyrat; 4,7-Dimethyloct-6-en-3-on; 2,6-Dimethylheptan-2-ol; 1-Methyl-4-(prop-1-en-2-yl)cyclohex-1-en; (E)-4-((3aS,7aS)-Hexahydro-1H-4,7-methanoinden-5(6H)-yliden)butanal; (E)-3-Methyl-5-(2,2,3-trimethylcyclopent-3-en-1-yl)pent-4-en-2-ol; Ethylhexanoat; Ethyloctanoat; (E)-3,7-Dimethylnona-1,6-dien-3-ol; (Z)-3,7-Dimethylnona-1,6-dien-3-yl-acetat; Ethylheptanoat; Ethyl-2,6,6-trimethylcyclohexa-1,3-dien-1-carboxylat; (1s,4s)-1,3,3-Trimethyl-2-oxa-bicyclo[2.2.2]octan; (2S)-1,3,3-Trimethylbicyclo[2.2.1]heptan-2-yl-acetat; (1S,2R,4R)-1,3,3-Trimethylbicyclo[2.2.1]heptan-2-ol); 1-(3,5,5,6,8,8-Hexa-methyl-5,6,7,8-tetrahydronaphthalin-2-yl)ethanon; 3-(4-Ethylphenyl)-2,2-dimethylpropanal; 3-(3-Isopropylphenyl)butanal; (3aR,6S,7a-S)-3a,4,5,6,7,7a-Hexahydro-1H-4,7-methanoinden-6-yl-propionat; 2,4,6-Trimethyl-4-phenyl-1,3-dioxan; 2-(sec-Butyl)cyclohexanon; (3aS,4S,7R,7aS)-Ethyl-Octahydro-1H-4,7-methanoinden-3a-carboxylat; 2-Methyldecannitril; 1-(3,3-Dimethylcyclohex-1-en-1-yl)pent-4-en-1-on; (3aR,6S,7aS)-3a,4,5,6,7,7a-hexahydro-1H-4,7-methanoinden-6-yl-isobutyrat; (E)-3,7-Dimethylocta-2,6-dien-1-ol; (E)-3,7-Dimethylocta-2,6-dien-1-yl-acetat; (E)-3,7-Dimethylocta-2,6-2-on; 1-(2,3,8,8-Tetramethyl-1,2,3,4,5,6,7,8-octahydronaphthalin-2-yl)ethanon); 2,4,6-Trimethylcyclohex-3-encarbaldehyd; 3,5,5-dien-1-yl-isobutyrat; Ethyl-2-Ethyl-6,6-dimethylcyclohex-2-encarboxylat; (E)-Oxacyclohexadec-12-en-2-on; Methyl-3-oxo-2-pentylcyclopentanacetat; (2S)-Ethyl-3-Isopropylbicyclo[2.2.1]hept-5-en-2-carboxylat; (Z)-hex-3-en-1-yl-butyrat; (E)-2-Benzylidenoctanal; Hexylisobutyrat; Hexyl-2-Hydroxybenzoat; 4,4a,5,9b-Tetrahydroindeno[1,2-d][1,3]dioxin; (E)-4-(2,6,6-Trimethylcyclohex-1-en-1-yl)but-3-en-2-on; (E)-4-(2,6,6-Trimethylcyclohex-2-en-1-yl)but-3-en-2-on; (E)-4-(2,5,6,6-Tetram-ethylcyclohex-2-en-1-yl)but-3-entrimethylhexylacetat; Isopropyl-2-methylbutanoat; (E)-3-Methyl-4-(2,6,6-trimethylcyclohex-2-en-1-yl)but-3-en-2-on; (3aR,6S,7aS)-3a,4,5,6,7,7a-Hexahydro-1H-4,7-methanoinden-6-yl-acetat; (Z)-3-Methyl-2-(pent-2-en-1-yl)cyclopent-2-enon; (Z)-3,4,5,6,6-Pentamethylhept-3-en-2-on; (Z)-1-(1-Ethoxyethoxy)hex-3-en; (2E,6Z)-3,7-Dimethylnona-2,6-diennitril; (Z)-hex-3-en-1-yl-Methylcarbonat; 3-(4-(tert-Butyl)phenyl)-2-methylpropanal; 3,7-Dimethylocta-1,6-dien-3-ol; 3,7-Dimethylocta-1,6-dien-3-yl-acetat; (4E)-9-Hydroxy-5,9-dimethyl-4-decenal; 2-Methyl-4-oxo-4H-pyran-3-yl-isobutyrat; Ethyl-2-methylpentanoat; 2,6-Dimethylhept-5-enal; 2-Isopropyl-5-methylcyclohexanol; 2-Isopropyl-5-methylcyclohexanon; 1-((15,8aS)-1,4,4,6-Tetramethyl-2,3,3a,4,5,8-hexahydro-1H-5,8a-methanoazulen-7-yl)ethanon; Undecan-2-on; Methyl-nicht-2-ynoat; 6,6-Dimethoxy-2,5,5-trimethylhex-2-en; 4-(4-Methylpent-3-en-1-yl)cyclohex-3-encarbaldehyd; 2-(2-(4-Methylcyclohex-3-en-1-yl)propyl)cyclopentanon; 2-Methyl-6-methyleneoct-7-en-2-yl-acetat; (E)-Methylnicht-2-enoat; (Z)-3,7,11-trimethyldodeca-1,6,10-trien-3-yl-acetat; (Z)-3,7-dimethylocta-2,6-dien-1-yl-acetat; 6,8-Dimethylnonan-2-ol; (Z)-Nicht-6-enal; 3-(4-Isobutyl-2-methylphenyl) propanal; 4-(tert-Pentyl)cyclohexanon; 2-Ethyl-N-methyl-N-(m-tolyl)butanamid; 2-Methyl-4-methylen-6-phenyltetrahydro-2H-pyran; 2-Cyclohexyliden-2-phenylacetonitril; 2-Cyclohexyliden-2-(o-tolyl)acetonitril; 2,2-Dimethyl-2-pheylethylpropanoat; 1-Methyl-4-(4-methylpent-3-en-1-yl)cyclohex-3-encarbaldehyd; 6-(sec-Butyl)chinolin; (E)-2-Ethyl-4-(2,2,3-trimethylcyclopent-3-en-1-yl)but-2-en-1-ol; 4-(4-Hydroxyphenyl)butan-2-on; 2,2,5-Trimethyl-5-pentylcyclopentanon; 2,2,2-trichlor-1-phenylethylacetat); ROSALVA (Dec-9-en-1-ol; (1-Methyl-2-(5-methylhex-4-en-2-yl)cyclopropyl)methanol; 4-Methylen-2-phenyltetrahydro-2H-pyran; 2-(1-(3,3-dimethylcyclohexyl)-ethoxy)-2-methylpropylcyclopropancarboxylat; 3-(4-Isobutylphenyl)-2-methylpropanal; 1-(Spiro[4.5]dec-6-en-7-yl)pent-4-en-1-on; (E)-5-Methylheptan-3-on-oxim; (E)-6-Ethyl-3-methyloct-6-en-1-ol; (E)-2-((3,5-Dimethylhex-3-en-2-yl)oxy)-2-methylpropylcyclopropancarboxylat; 1-Methyl-4-propan-2-ylcyclohexa-1,4-dien; 1-Methyl-4-(propan-2-ylidene)cyclohex-1-en; 2-(4-Methylcyclohex-3-en-1-yl)propan-2-yl-acetat; 3,7-Dimethyloctan-3-ol; 2,6-Dimethyloctan-2-ol; Oxacyclohexadecan-2-on; (E)-Tridec-2-ennitril; (E)-4-Methyldec-3-en-5-ol; 2,2,5-Trimethyl-5-pentylcyclopentanon; (2,2-Dimethoxyethyl)benzol und 2-(2,4-Dimethylcyclohexyl)pyridin) ausgewählt ist.

13. Verkapselte Zusammensetzung, die durch das Verfahren nach einem der Ansprüche 1 bis 12 erhältlich ist.

14. Konsumprodukt, das die verkapselte Zusammensetzung nach Anspruch 13 umfasst, wobei es sich bei dem Konsumprodukt um ein Textilpflegeprodukt, ein Haushaltspflegeprodukt oder ein Körperpflegeprodukt handelt.

15. Verwendung der verkapselten Zusammensetzung nach Anspruch 13 zum Erhalten eines Konsumprodukts.

## Revendications

1. Procédé d'obtention d'une composition encapsulée comprenant au moins une microcapsule noyau-enveloppe cationique, dans lequel l'au moins une microcapsule noyau-enveloppe cationique comprend un noyau comprenant au moins un matériau fonctionnel et une enveloppe entourant le noyau, dans laquelle l'enveloppe comprend une résine formée par réaction d'au moins un isocyanate araliphatique trifonctionnel avec au moins un précondensat amino-aldéhyde partiellement alkylé, le procédé comprenant les étapes de :

   a) Fourniture d'une composition de noyau ;
   b) Fourniture d'une phase aqueuse comprenant l'au moins un précondensat amino-aldéhyde partiellement alkylé et un polymère stabilisant cationique ;
   c) Émulsification de la composition de noyau fournie à l'étape a) avec la phase aqueuse fournie à l'étape b) afin d'obtenir une émulsion de gouttelettes de composition de noyau ayant une taille moyenne en volume de 1 à 100 $\mu$m, de préférence de 5 à 50 $\mu$m, encore plus préférablement de 7 à 20 $\mu$m ;
   d) Chauffage de l'émulsion obtenue à l'étape c) jusqu'à une température de 70 à 95 °C, de préférence de 80 à 90 °C ; de préférence à un pH de 2,0 à 4,5, plus préférablement de 2,5 à 4,0, encore plus préférablement de 3,0 à 3,8, encore plus préférablement de 3,4 à 3,6, afin d'obtenir des microcapsules noyau-enveloppe ;
   e) Éventuellement : ajout d'un agent piégeant le formaldéhyde ; et
   f) Le fait de laisser refroidir le mélange obtenu à l'étape d) ou l'étape e) jusqu'à température ambiante, afin d'obtenir une suspension de microcapsules ;

   dans lequel l'au moins un isocyanate araliphatique trifonctionnel est ajouté à la phase aqueuse ou à l'émulsion avant, pendant ou après l'étape c).

2. Procédé selon la revendication 1, dans lequel l'au moins un isocyanate araliphatique trifonctionnel est un adduit d'un triol aliphatique avec trois diisocyanates araliphatiques ; éventuellement un adduit de 2-éthylpropane-1,2,3-triol ou de 2-éthyl-2-(hydroxyméthyl)propane-1,3-diol avec un composé choisi dans le groupe constitué par 1-isocyanato-2-(isocyanatométhyl)benzène, 1-isocyanato-3-(isocyanatométhyl)benzène et 1-isocyanato-4-(isocyanatométhyl)benzène ; éventuellement un adduit de 2-éthylpropane-1,2,3-triol avec 1-isocyanato-3-(isocyanatométhyl)benzène.

3. Procédé selon l'une des revendications 1 ou 2, dans lequel l'au moins un précondensat amino-aldéhyde partiellement alkylé est choisi dans le groupe constitué par des précondensats de polyméthylol-1,3,5-triamino-2,4,6-triazine partiellement méthylés, une polyméthylolurée partiellement méthylée, une polyméthylol-benzoguanamine partiellement méthylée et un polyméthylol-glycouril partiellement méthylé, plus préférablement des précondensats de polyméthylol-1,3,5-triamino-2,4,6-triazine partiellement méthylés.

4. Procédé selon la revendication 3, dans lequel le précondensat de polyméthylol-1,3,5-triamino-2,4,6-triazine partiellement méthylé peut être obtenu en faisant réagir de la mélamine, du formaldéhyde et du méthanol, et dans lequel le rapport molaire de la mélamine sur le formaldéhyde est de 1:3 à 1:6 et le rapport molaire de la mélamine sur l'alcool est 1:2 à 1:4.

5. Procédé selon l'une des revendications 1 à 4, dans lequel le rapport pondéral de l'au moins un isocyanate araliphatique trifonctionnel sur l'au moins un précondensat amino-aldéhyde partiellement alkylé est 0,25 à 0,50, de préférence 0,3 à 0,4.

6. Procédé selon l'une des revendications 1 à 5, dans lequel le polymère stabilisant cationique est un copolymère pouvant être obtenu par copolymérisation de chlorure de méthacrylamidopropyltriméthylammonium, d'acide acrylique ou d'acide méthacrylique et, éventuellement, d'acrylamide.

7. Procédé selon la revendication 6, dans lequel le copolymère stabilisant cationique est un copolymère de 2 à 99 % en moles, plus particulièrement de 30 à 95 % en moles, encore plus particulièrement de 60 à 90 % en moles, de chlorure de méthacrylamidopropyltriméthylammonium (MAPTAC) ; et de 1 à 98 % en moles, plus particulièrement de 5 à 70 % en moles, encore plus particulièrement de 10 à 40 % en moles, d'acide acrylique, et de 0 à 50 % en moles, plus particulièrement de 0,1 à 5 % en moles, d'acrylamide.

8. Procédé selon l'une des revendications 1 à 7, comprenant en outre l'étape d'ajout, entre l'étape d) et l'étape f), de préférence entre l'étape e) et l'étape f), d'un agent de suspension.

9. Procédé selon la revendication 8, dans lequel l'agent de suspension est un polymère cationique, de préférence un copolymère cationique.

10. Procédé selon l'une des revendications 1 à 9, comprenant en outre l'étape d'ajout d'au moins un conservateur au mélange obtenu à l'étape d) ou à la suspension de microcapsules obtenue à l'étape f).

11. Procédé selon l'une des revendications 1 à 10, dans lequel la composition de noyau comprend au moins une matière fonctionnelle choisie dans le groupe constitué par les ingrédients de fragrance, les ingrédients cosmétiques, les ingrédients biologiquement actifs et les renforçateurs de substrat.

12. Procédé selon la revendication 11, dans lequel la composition de noyau comprend au moins un ingrédient de fragrance choisi dans le groupe constitué par 2,6,10-triméthylundéc-9-énal ; acétate de 2-(tert-butyl)cyclohexyle ; décanal ; 2-méthyldécanal ; undéc-10-énal) ; undécanal ; dodécanal ; 2-méthylundécanal ; (E)-undéc-9-énal ; (E)-dodéc-2-énal ; 2-(isopentyloxy)acétate d'allyle ; 3-cyclohexylpropanoate d'allyle ; heptanoate d'allyle ; 1-((2-(tert-butyl)cyclohexyl)oxy)-butan-2-ol ; 1,3,4,5,6,7-hexahydro-$\beta$-,1,1,5,5-pentaméthyl-2H-2,4a-méthanonaphtalène-8-éthanol ; 2-hydroxybenzoate de pentyle ; formiate de 1-(3,3-diméthylcyclohexyl)éthyle ; (1R,2S,4R)-2'-isopropyl-1,7,7-triméthyl-spiro[bicyclo[2.2.1]heptane-2,4'-[1,3]dioxane] ; 8-(sec-butyl)-5,6,7,8-tétrahydroquinoléine) ; (éthoxyméthoxy)-cyclododécane ; (1S,2R,5R)-2-éthoxy-2,6,6-triméthyl-9-méthylène-bicyclo[3.3.1]nonane ; acétate de (2S,4S)-1,7,7-triméthyl-bicyclo[2.2.1]-heptan-2-yle ; butyrate de 1-butoxy-1-oxopropan-2-yle ; acétate de 4-(tert-butyl)cyclohexyle ; (Z)-4,11,11-triméthyl-8-méthylène-bicyclo[7.2.0]-undéc-4-ène ; 1,1,2,3,3-pentaméthyl-2,3,6,7-tétrahydro-1H-indén-4(5H)-one ; 5-tert-butyl-2-méthyl-5-propyl-2H-furane ; (E)-3,7-diméthylocta-2,6-diénal ; (E)-3,7-diméthylocta-2,6-diénal ; (Z)-1,1-diéthoxy-3,7-diméthylocta-2,6-diène ; 3,7-diméthyloct-6-énal ; 3,7-diméthyloct-6-én-1-ol acétate de 3,7-diméthyloct-6-én-1-yle ; formiate de 3,7-diméthyloct-6-én-1-yle ; 3,7-diméthyloct-6-ènenitrile ; propionate de 3,7-diméthyloct-6-én-1-yle ; dodécanenitrile ; 4-cyclohexyl-2-méthylbutan-2-ol ; (Z)-3-méthylcyclotétradéc-5-énone ; 3-(4-isopropylphényl)-2-méthylpropanal ; 2-(cyclohexyloxy)acétate d'allyle ; 2-hydroxybenzoate de cyclohexyle ; 8,8-diméthyl-1,2,3,4,5,6,7,8-octahydronaphtalène-2-carbaldéhyde ; (E)-1-(2,6,6-triméthylcyclohexa-1,3-dién-1-yl)but-2-én-1-one ; (E)-1-(2,6,6-triméthylcyclohex-2-én-1-yl)but-2-én-1-one ; (E)-1-(2,6,6-triméthyl-cyclohex-3-én-1-yl)but-2-én-1-one ; (E)-déc-4-énal ; 2-pentylcyclopentanone ; acide propanedioïque ester de 1-(1-(3,3-diméthylcyclohexyl)éthyl) 3-éthyle ; 3-méthyl-2-pentylcyclopent-2-énone ; 2-méthyl-1-phénylpropan-2-ol ; acétate de 2-méthyl-1-phénylpropan-2-yle ; butyrate de 2-méthyl-1-phénylpropan-2-yle ; 4,7-diméthyloct-6-én-3-one ; 2,6-diméthylheptan-2-ol ; 1-méthyl-4-(prop-1-én-2-yl)cyclohex-1-ène ; (E)-4-((3aS,7aS)-hexahydro-1H-4,7-méthanoindén-5(6H)-ylidène)butanal ; (E)-3-méthyl-5-(2,2,3-triméthylcyclopent-3-én-1-yl)pent-4-én-2-ol ; hexanoate d'éthyle ; octanoate d'éthyle ; (E)-3,7-diméthylnona-1,6-dién-3-ol ; acétate de (Z)-3,7-diméthylnona-1,6-dién-3-yle ; heptanoate d'éthyle ; 2,6, 6-triméthylcyclohexa-1,3-diène-1-carboxylate d'éthyle ; (1s,4s)-1,3,3-triméthyl-2-oxa-bicyclo[2.2.2]octane ; acétate de (2S)-1,3,3-triméthylbicyclo[2.2.1]heptan-2-yle ; (1S,2R,4R)-1,3,3-triméthylbicyclo[2.2.1]heptan-2-ol) ; 1-(3,5,5,6,8,8-hexaméthyl-5,6,7,8-tétrahydronaphtalén-2-yl)éthanone ; 3-(4-éthylphényl)-2,2-diméthylpropanal ; 3-(3-isopropylphényl)butanal ; propionate de (3aR,6S,7aS)-3a,4,5,6,7,7a-hexahydro-1H-4,7-méthanoindén-6-yle ; 2,4,6-triméthyl-4-phényl-1,3-dioxane ; 2-(sec-butyl)cyclohexanone ; octahydro-1H-4,7-méthanoindène-3a-carboxylate d'éthyle (3aS,4S,7R,7aS) ; 2-méthyldécanenitrile ; 1-(3,3-diméthylcyclohex-1-én-1-yl)pent-4-én-1-one ; (3aR,6S,7aS)-3a,4,5,6,7,7a-hexahydro-1H-4,7-méthanoindén-6-yle isobutyrate ; (E)-3,7-diméthylocta-2,6-dién-1-ol ; (E)-3,7-diméthylocta-2,6-dién-1-yle acétate ; (E)-3,7-diméthylocta-2,6-2-one ; 1-(2,3,8,8-tétraméthyl-1,2,3,4,5,6,7,8-octahydronaphtalén-2-yl)éthanone) ; 2,4,6-triméthylcyclohex-3-ènecarbaldéhyde ; 3,5,5-dién-1-yle isobutyrate ; 2-éthyl-6,6-diméthylcyclohex-2-ènecarboxylate d'éthyle ; (E)-oxacyclohexadéc-12-én-2-one ; 3-oxo-2-pentylcyclopentaneacétate de méthyle ; 3-isopropylbicyclo[2.2.1] hept-5-ène-2-carboxylate d'éthyle (2S) ; butyrate d'hex-3-én-1-yle (2) ; 2-benzylidèneoctanal (E) ; isobutyrate d'hexyle ; 2-hydroxybenzoate d'hexyle ; 4,4a,5,9b-tétrahydroindéno[1,2-d][1,3]dioxine ; 4-(2,6,6-triméthylcyclohex-1-én-1-yl)but-3-én-2-one (E) ; 4-(2,6,6-triméthylcyclohex-2-én-1-yl)but-3-én-2-one (E) ; (E)-4-(2,5,6,6-tétraméthylcyclohex-2-én-1-yl)but-3-én-triméthylhexyle acétate ; 2-méthylbutanoate d'isopropyle ; (E)-3-méthyl-4-(2,6,6-triméthylcyclohex-2-én-1-yl)but-3-én-2-one ; (3aR,6S,7aS)-3a,4,5,6,7,7a-hexahydro-1H-4,7-méthanoindén-6-yle acétate ; (Z)-3-méthyl-2-(pent-2-én-1-yl)cyclopent-2-énone ; (Z)-3,4,5,6,6-pentaméthylhept-3-én-2-one ; (Z)-1-(1-éthoxyéthoxy)hex-3-ène (2E,6Z)-3,7-diméthylnona-2,6-diènenitrile ; carbonate de méthyle de (2)-hex-3-én-1-yle ; 3-(4-(tert-butyl)phényl)-2-méthylpropanal ; 3,7-diméthylocta-1,6-dién-3-ol ; acétate de 3,7-diméthylocta-1,6-dién-3-yle ; (4E)-9-hydroxy-5,9-diméthyl-4-décénal ; isobutyrate de 2-méthyl-4-oxo-4H-pyran-3-yle ; 2-méthylpentanoate d'éthyle ; 2,6-diméthylhept-5-énal ; 2-isopropyl-5-méthylcyclohexanol ; 2-isopropyl-5-méthylcyclohexanone ; 1-((1S,8aS)-1,4,4,6-1-étraméthyl-2,3,3a,4,5,8-hexahydro-1H-5,8a-méthanoazulén-7-yl)éthanone ; undécan-2-one ; non-2-ynoate de méthyle ; 6,6-diméthoxy-2,5,5-triméthylhex-2-ène ; 4-(4-méthylpent-3-én-1-yl)cyclohex-3-ènecarbaldéhyde ; 2-(2-(4-méthylcyclohex-3-én-1-yl)propyl)cyclopentanone ; acétate de 2-méthyl-6-méthylèneoct-7-én-2-yle ; (E)-non-2-énoate de méthyle ; acétate de (Z)-3,7,11-triméthyldodéca-1,6,10-trién-3-yle ; acétate de (Z)-3,7-diméthylocta-2,6-dién-1-yle ; 6,8-diméthylnonan-2-ol ; (Z)-non-6-énal ; 3-(4-isobutyl-2-méthylphényl) propanal ; 4-(tert-pentyl)cyclohexanone ; 2-éthyl-N-méthyl-N-(m-tolyl)butanamide ; 2-méthyl-4-méthylène-6-phényltétrahydro-2H-pyrane ; 2-cyclohexylidène-2-phénylacétonitrile ; 2-cyclohexylidène-2-(o-tolyl)acétonitrile ; propanoate de 2,2-diméthyl-2-phényléthyle ; 1-méthyl-4-(4-méthylpent-3-én-1-yl)cyclohex-3-ènecarbaldéhyde ; 6-(sec-butyl)quinoléine ; (E)-2-éthyl-4-(2,2,3-triméthylcyclopent-3-én-1-yl)but-2-én-1-ol ; 4-(4-hydroxyphényl)butan-2-one ; 2,2,5-triméthyl-5-pentylcyclopentanone ; acétate de 2,2,2-trichloro-1-phényléthyle) ROSALVA (déc-9-én-1-ol ; (1-méthyl-2-(5-méthylhex-4-én-2-yl)cyclopropyl)-méthanol ; 4-méthylène-2-phényltétrahydro-2H-pyrane ; 2-(1-(3,3-diméthylcyclohexyl)-éthoxy)-2-méthylpropyl cyclopropanecarboxylate ; 3-(4-isobutylphényl)-2-méthylpropanal ; 1-(spiro[4.5]déc-6-én-7-yl)pent-4-én-1-one ; (E)-5-méthylheptan-3-one oxime ; (E)-6-éthyl-3-méthyloct-6-én-1-ol ; cyclopropanecarboxylate de (E)-2-((3,5-diméthylhex-3-én-2-yl)oxy)-2-méthylpropyle ; 1-méthyl-4-propan-2-ylcyclohexa-1,4-diène ; 1-méthyl-4-(propan-2-ylidène)cyclohex-1-ène ; acétate de 2-(4-méthylcyclohex-3-én-1-yl)propan-2-yle ; 3,7-diméthyloctan-3-ol ; 2,6-diméthyloctan-2-ol ; oxacyclohexadécan-2-one ; (E)-tridéc-2-ènenitrile ; (E)-4-méthyldéc-3-én-5-ol ; 2,2,5-triméthyl-5-pentylcyclopentanone ; (2,2-diméthoxyéthyl)benzène et 2-(2,4-diméthylcyclohexyl)pyridine).

**13.** Composition encapsulée pouvant être obtenue par le procédé selon l'une des revendications 1 à 12.

**14.** Produit de consommation comprenant la composition encapsulée selon la revendication 13, dans lequel le produit de consommation est un produit d'entretien de tissus, un produit d'entretien ménager ou un produit de soin personnel.

**15.** Utilisation de la composition encapsulée selon la revendication 13 pour obtenir un produit de consommation.

**EP 4 139 041 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2008098387 A1
- WO 2016207180 A1
- WO 2015110568 A1
- US 2020087598 A1
- WO 2019170528 A1

### Non-patent literature cited in the description

- *CHEMICAL ABSTRACTS*, 94089-18-6
- *CHEMICAL ABSTRACTS*, 20770-40-5
- *CHEMICAL ABSTRACTS*, 100403-19-8
- **A.W. ISLAM ; A. ZAVVADI ; V.N. KABADI**. *Chem. Process Eng.*, 2012, vol. 33, 243-253